# FASCICULE DE BREVET EUROPEEN

(11) **EP 1 430 074 B1**
(45) Date de publication et mention de la délivrance du brevet: **06.05.2009**
(21) Numéro de dépôt: 02796324.8
(22) Date de dépôt: 29.08.2002
(51) Int. Cl.: C07J 41/00, A61K 9/127, A61K 31/575

(54) **DERIVES LIPIDIQUES D'AMINOGLYCOSIDES-POUR LA TRANSFECTION**
LIPID-AMINOGLYKOSID KONJUGATE ZUR TRANSFEKTION
AMINOGLYCOSIDE LIPID DERIVATIVES FOR TRANSFECTION

(30) Priorité: 29.08.2001 FR 0111199; 16.11.2001 US 331452 P
(43) Date de publication de la demande: 23.06.2004
(73) Titulaire: Aventis Pharma S.A., 92160 Antony (FR); CENTRE NATIONAL DE LA RECHERCHE SCIENTIFIQUE, 75794 Paris Cedex 16 (FR); INSTITUT NATIONAL DE LA SANTE ET DE LA RECHERCHE MEDICALE, 75654 Paris Cédex 13 (FR)
(72) Inventeur: LEHN, Jean-Marie, F-67000 Strasbourg (FR); LEHN, Pierre, F-75019 Paris (FR); VIGNERON, Jean-Pierre, F-91790 Boissy sur Saint Yon (FR)
(74) Mandataire: Warcoin, Jacques
(86) Numéro de dépôt international: PCT/FR2002/002959
(87) Numéro de publication internationale: WO 2003/018603

(56) Documents cités:
- WO-A-00/40692
- WO-A-01/48233
- WO-A-96/24334
- WO-A-98/55490
- WO-A-99/12945

## Description

La présente invention est relative à de nouveaux composés qui permettent le transfert d'acides nucléiques dans des cellules. Plus précisément, ces nouveaux composés sont des dérivés lipidiques d'aminoglycosides. Ils sont utiles pour la transfection *in vitro*, *ex vivo* ou *in vivo* d'acides nucléiques dans différents types cellulaires.

Avec le développement des biotechnologies, il est devenu nécessaire de transférer efficacement des acides nucléiques dans les cellules. Il peut s'agir du transfert d'acides nucléiques dans des cellules *in vitro,* par exemple pour la production de protéines recombinantes, ou au laboratoire, pour l'étude de la régulation de l'expression de gènes, le clonage de gènes, ou toute autre manipulation impliquant l'ADN. Il peut également s'agir du transfert d'acides nucléiques dans des cellules *in vivo,* par exemple pour la création d'animaux transgéniques, la réalisation de vaccins, des études de marquage ou également des approches thérapeutiques. Il peut encore s'agir du transfert d'acides nucléiques dans des cellules *ex vivo,* dans des approches de greffes de moelle osseuse, d'immunothérapie ou d'autres méthodes impliquant le transfert de gènes dans des cellules prélevées d'un organisme, en vue de leur réadministration ultérieure.

Aujourd'hui, plusieurs méthodes sont proposées pour la délivrance intracellulaire de matériel génétique exogène. L'une d'entre elles, en particulier, repose sur l'emploi de vecteurs non-viraux qui constitue une alternative très avantageuse par rapport aux méthodes virales qui ne sont pas complètement dénuées de risques. Ces vecteurs synthétiques ont deux fonctions principales : complexer et compacter l'acide nucléique à transfecter, et promouvoir son passage à travers la membrane plasmique et éventuellement à travers l'enveloppe nucléaire.

Plusieurs familles de vecteurs synthétiques ont ainsi été développées, comme par exemple les polymères ou encore les vecteurs biochimiques (constitués d'une protéine cationique associée à un récepteur cellulaire), mais un progrès important a surtout été accompli avec le développement des lipofectants, et plus particulièrement des lipides cationiques. Il a ainsi été mis en évidence que les lipides cationiques, du fait de leur charge globale positive, interféraient spontanément avec l'ADN globalement négatif, formant des complexes nucléolipidiques capables à la fois de protéger l'ADN vis-à-vis des nucléases et de se fixer sur les membranes cellulaires pour une libération intracellulaire de l'ADN. Ainsi, la plupart des vecteurs non-viraux mis au point à ce jour sont basés sur la protonation d'amines pour permettre la liaison à l'ADN.

Différentes sortes de lipides cationiques ont été synthétisées à ce jour : des lipides comportant un groupement ammonium quaternaire (par exemple le DOTMA, DOTAP, DMRIE, DLRIE...), des lipopolyamines comme par exemple le DOGS, le DC-Chol ou les lipopolyamines divulguées dans la demande de brevet WO 97/18185, des lipides associant à la fois un groupement ammonium quaternaire et une polyamine comme le DOSPA, ou encore des lipides comportant diverses autres entités cationiques dérivées des amines, notamment des groupes amidinium (par exemple l'ADPDE, ADODE ou les lipides du brevet EP 0 888 379 B1 ou US 6,143,729, en particulier le BGTC).

Bien que les relations structure/activité des lipides cationiques ne soient pas encore vraiment comprises, on considère généralement que la nature de la partie cationique a un impact majeur sur l'activité de transfection, bien que ce soit la nature et les propriétés physico-chimiques de la molécule entière (tel que 1a balance hydrophobie/hydrophilie, les interactions avec des co-lipides etc...) qui déterminent l'efficacité d'un lipide cationique donné in fine (voir par exemple Felgner et al., Am. Chem. Soc., 1996, pp. 177-190).

C'est pourquoi, ces dernières années, de nombreux efforts ont été déployés pour développer des lipides cationiques ayant de nouvelles entités cationiques. La présente invention a précisément pour objet de proposer de nouveaux composés transfectants, originaux de par leur partie cationique constituée d'aminoglycosides ou de leurs dérivés polyguanidylés, qui sont susceptibles d'être utilisés efficacement pour la transfection *in vitro, ex vivo* ou *in vivo* d'acides nucléiques. Ces nouveaux composés sont particulièrement intéressants car les aminoglycosides peuvent interagir avec des substructures tridimensionnelles trouvées dans une grande variété de molécules d'ARN (Walter et al., Curr. Opin. Chem. Biol., 1999, 3, pp. 694-704) et ils constituent donc des nouveaux groupes cationiques ayant des caractéristiques structurelles particulièrement bien adaptées pour la synthèse d'une grande variété de composés transfectants. En effet, les aminoglycosides sont des composés multifonctionnels contenant jusqu'à 6 groupes aminés et plusieurs fonctions hydroxyles. Une acylation sélective d'un ou plusieurs de ces groupes a donc permis d'obtenir des aminoglycosides ayant les propriétés lipophiles requises pour une utilisation en transfection. En outre, les aminoglycosides peuvent être facilement polyguanidylés en une seule étape, ce qui permet par exemple de synthétiser des composés transfectants présentant des groupes guanidinium dans leur partie cationique à la place des amines. Enfin, ces nouveaux composés transfectants sont également particulièrement intéressants d'un point de vue pharmaceutique car les aminoglycosides qui constituent leur partie cationique sont des composés naturels déjà largement utilisés en pharmacie comme antibiotiques, leur activité antibactérienne étant en fait dérivée de leur interaction avec les ARNr procaryotes (Moazed et al., Nature, 1987, 327, pp. 389-394 ; Puhrohit et al., Nature, 1994, 370, pp. 659-662).

La présente invention a ainsi pour premier objet des composés transfectants caractérisés en ce qu'ils sont constitués d'un aminoglycoside relié à un lipide par l'intermédiaire d'un espaceur, de formule générale (I) :

A-Y-L (I)

dans laquelle :
- A représente un aminoglycoside défini ci-dessous,
- Y représente un espaceur, et
- L représente :
   - soit un radical -(R₁)R₂ avec R₁ et R₂ qui représentent, indépendamment l'un de l'autre, un atome d'hydrogène ou une chaîne aliphatique grasse ou R₁ ou R₂ est absent, étant entendu que l'un au moins de R₁ et de R₂ représente une chaîne aliphatique grasse défini ci dessous.
   - soit un radical N-R₃ ou -O-R₃, avec R₃ qui représente un dérivé stéroïdien défini ci dessous.

Au sens de la présente invention, on entend par « aminoglycoside » (ou « aminosides ») l'amikacine, l'arbékacine, la dibékacine, la gentamicine, l'isépamycine, la kanamycine, la micronomycine, la paromomycine, la néomycine, ou la tobramycine

Selon la présente invention, on entend par « espaceur » tout groupe chimique permettant à la fois d'assurer la liaison entre l'aminoglycoside ou son dérivé polyguanidylé et la partie lipidique de la molécule, et d'éloigner ces deux parties afin d'atténuer toute interaction non-désirée entre elles. Par conséquent, l'espaceur est un groupe chimique bifonctionnel qui peut par exemple être constitués d'une ou plusieurs fonctions chimiques choisies parmi les alkyles ayant 1 à 6 atomes de carbone, les fonctions cétone, ester, éther, amino, amide, amidine, carbamate, thiocarbamate, le glycérol, l'urée, la thiourée, ou encore les cycles aromatiques. Par exemple, l'espaceur peut être choisi parmi les radicaux de formule :

-C(O)-

-NH-C(O)-CH₂-CH₂-

-W-(CH₂-)ₖ-W'-

ou encore :

-(CH₂-)ᵢ-W-(CH₂)ⱼ-

dans laquelle i, j et k sont des entiers choisis entre 1 et 6 inclus et W et W' sont des groupes identiques ou différents choisis parmi les fonctions cétone, ester, éther, amino, amide, amidine, carbamate, thiocarbamate, le glycérol, l'urée, la thiourée, ou encore les cycles aromatiques,

Au sens de la présente invention, on entend par « chaînes aliphatiques grasses » des radicaux alkyle contenant 1, 2 ou 3 insaturation et comprenant 14, 16, 18, 20 ou 22 atomes de carbone. On peut citer plus particulièrement les radicaux aliphatiques (CH₂)₁₃CH₃, (CH₂)₁₅CH₃ et -(CH₂)₁₇CH₃.

Au sens de la présente invention, on entend par « dérivés stéroïdiens » les composés polycycliques de type cholestane. Ces composés peuvent être naturels ou non et sont de préférence choisis parmi le cholestérol, le cholestanol, le 3-α-5-cycle-5-α,-cholestan-6-β-ol, l'acide cholique, le cholestérylformiate, le chotestanylformiate, le 3α,5-cyclo-5α-cholestan-6β-yl formiate, la cholestérylamine, la 6-(1,5-diméthylhexyl)-3a,5a-diméthylhexadécahydrocyclopenta[a]cyclopropa[2,3]-cyclopenta[1,2-f]naphta-lèn-10-ylamine, ou la cholestanylamine.

Les composés transfectants pour lesquels A et Y sont tels que définis précédemment dans la formule générale (I) et L est un radical -OR₃, avec R₃ qui représente un dérivé stéroïdien constituent une sous-classe de composés transfectants préférés au sens de la présente invention.

Encore plus préférentiellement, les composés transfectants selon la présente invention sont choisis parmi les composés de formule générale (I) pour lesquels A et Y sont tels que définis précédemment et L est un radical -OR₃, avec R₃ qui représente un radical cholestérol.

Des composés transfectants encore plus particulièrement préférés sont notamment le 3β[6'-kanamycin-carbamoyl]cholestérol (« KanaChol ») de formule : 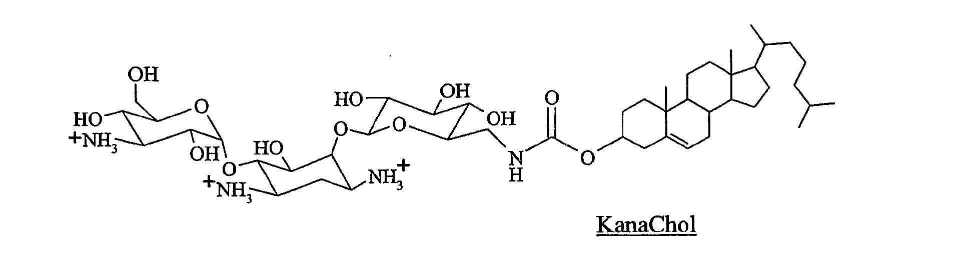 le 3β[6'-(1,3,3"-triguanidino)kanamycin-carbamoyl]cholestérol (« TGKC » ) de formule : 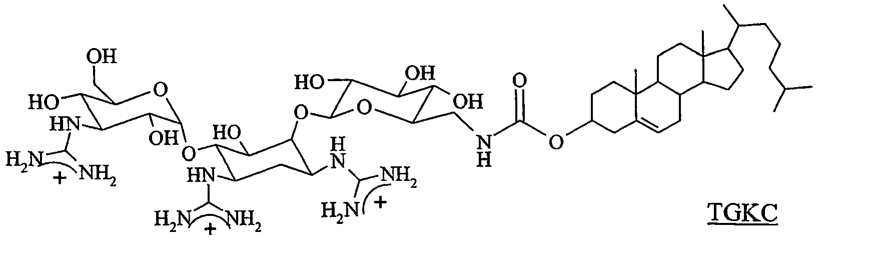 et le (5"-aminoethylsulfanyl) neomycin carbamoylcholestérol (« NéoChol ») de formule :

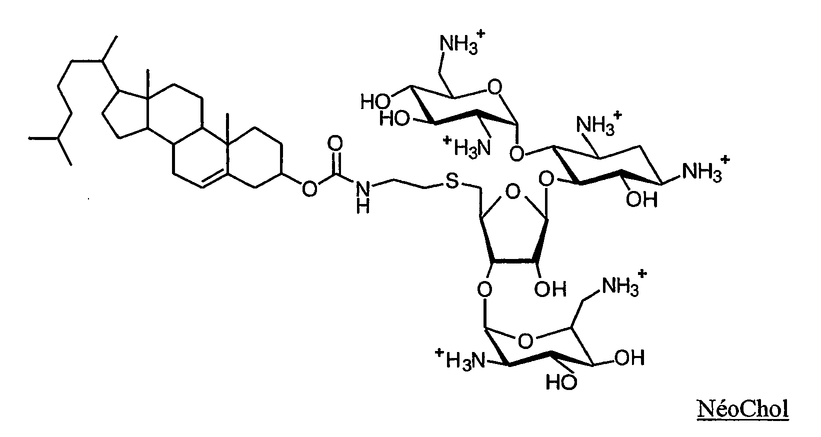

Il est entendu que la présente invention concerne également les produits de formule générale (I), ainsi que leurs mélanges, ou leurs sels.

Notamment, les composés de l'invention peuvent se présenter sous forme de sels non toxiques et pharmaceutiquement acceptables. Ces sels non toxiques comprennent les sels avec les acides minéraux (par exemple l'acide chlorhydrique, sulfurique, bromhydrique, phosphorique, nitrique), ou avec les acides organiques (acide acétique, propionique, succinique, maléique, hydroxymaléique, benzoïque, fumarique, méthanesulfonique trifluoroacétique ou oxalique).

La préparation des composés de formule générale (I) selon la présente invention s'effectue en mettant en oeuvre les étapes suivantes, dans l'ordre présenté ou selon toute autre variante connue et également adaptée, en utilisant les techniques de synthèse organique classique, en solution ou sur des supports solides, bien connues de l'homme du métier :

### 1) Obtention de la partie lipidique L

Lorsque la partie lipidique L des composés de formule générale (I) est représentée par un radical -(R₁)R₂ rattaché par exemple à un atome d'azote de l'espaceur, on forme tout d'abord l'amine de formule HN(R₁)R2 . Ladite amine peut être obtenue par condensation d'un acide carboxylique et d'une amine contenant l'un le substituant R₁ et l'autre le substituant R₂ pour former l'amide correspondant, suivie de la réduction de dudit amide ainsi obtenu.

La formation de l'amide est avantageusement effectuée par mélange des constituants et fusion par chauffage à une température supérieure à la température de fusion des substances impliquées, en général entre 20°C et 200°C, puis élimination de l'eau produite par déshydratation du milieu; ou plus avantageusement en présence d'un agent dessicant tel que par exemple le pentoxyde de phosphore ou toute autre substance pouvant absorber l'eau. La formation de cet amide intermédiaire peut également se faire en employant une variante de cette méthode ou toute autre méthode de formation d'amide (tel que par exemple le couplage de type peptidique) impliquant les acides carboxyliques ou leurs dérivés, des conditions et des réactifs variés [R.C. Larock, Comprehensive Organic Transformations, VCH Editeurs] et bien connues de l'homme de l'art.

La réduction de l'amide précédemment obtenu en amine de formule HN(R₁)R₂ peut être effectuée par exemple en utilisant un réducteur tel que l'hydrure double de lithium et d'aluminium, ou tout autre hydrure ou réducteur efficace dans ce cas. On opère alors de préférence dans un solvant non protique (par exemple le tétrahydrofurane ou encore les éthers), à température inférieure à la température d'ébullition du solvant et sous atmosphère sèche et/ou inerte.

Selon une autre variante, la partie lipidique désignée comme HN(R₁)R₂ peut être disponible commercialement.

Lorsque la partie lipidique L des composés de formule générale (I) est représentée par un radical -NR₃ ou -OR₃, celle-ci est de préférence choisie parmi les produits disponibles dans le commerce.

### 2) Greffage de l'espaceur Y

L'espaceur Y est ensuite fixé à la partie lipidique L obtenue à l'étape précédente selon les techniques classiques connue de l'homme de l'art. Selon une variante préférée, une liaison amide est réalisée par *N-*acylation de la partie lipidique L dans un solvant approprié tel que le dichlorométhane, le chloroforme, le tétrahydrofurane, ou tout autre éther, à température inférieure à la température d'ébullition du solvant, et sous atmosphère sèche et/ou inerte. Cette réaction se déroule de préférence en présence d'une base aminée comme la *N,N-*diméthylaminopyridine, ou en présence de cette base mélangée avec des bases aminées non-nucléophiles comme la triéthylamine ou encore l'éthyldiisopropylamine. La pyridine peut également être employée, seule ou en mélange avec une autre base, diluée par un des solvants cités ou utilisée elle-même comme solvant.

Selon une autre variante, quand la partie lipidique L représente un radical - OR₃, le traitement par le phosgène de l'alcool correspondant R₃OH donne un chloroformiate (parfois disponible dans le commerce).

### 3) greffage de l'aminoglycoside

Le greffage de l'aminoglycoside sur l'ensemble Y-L se fait en utilisant les fonctions amines et/ou hydroxyles portées par l'aminoglycoside, qu'elles soient présentes dans le composé naturel ou qu'elles proviennent d'une modification réalisée selon les méthodes classiques connues de l'homme de l'art.

Selon une variante, une liaison amide est réalisée par N-acylation d'une fonction amine de l'aminoglycoside par l'ensemble Y-L convenablement activé selon les méthodes classiques utilisées pour créer une liaison peptidique.

Lorsque la partie lipidique L des composés de formule générale (I) est représentée par le radical -OR₃, une possibilité préférée consiste à réunir les parties A et L par l'intermédiaire d'une liaison carbamate obtenue en traitant l'aminoglycoside par le chloroformiate , parfois disponible commercialement, de l'alcool R₃OH. Cette réaction a lieu dans un solvant approprié tel que le dichlorométhane, le chloroforme, le tétrahydrofurane, le diméthylformamide ou tout autre éther, à une température inférieure à la température d'ébullition du solvant, sous atmosphère sèche et/ou inerte et en présence d'une base aminée non-nucléophile telle que la triéthylamine, l'éthyldiisopropylamine ou la pyridine.

D'une manière générale, les aminoglycosides utilisés préférentiellement dans le cadre de l'invention sont tous disponibles commercialement.

Naturellement, lorsque les différents substituants peuvent interférer avec la réaction, il est préférable de les protéger préalablement avec des radicaux compatibles et pouvant être mis en place et éliminés sans toucher au reste de la molécule. On opère pour cela selon les méthodes classiques connues de l'homme du métier, et notamment selon les méthodes décrites dans T. W. Greene, Protective Groups in Organic Synthesis, Wiley-Interscience, dans McOmie, Protective Groups in Organic Chemistry, Plenum Press, ou dans P. J. Kocienski, Protecting Groups, Thieme.

Par ailleurs, chaque étape du procédé de préparation peut être suivie, le cas échéant, des étapes de séparation et de purification du composé obtenu selon toute méthode connue de l'homme du métier.

Un autre objet de l'invention concerne les compositions comprenant un composé transfectant selon l'invention et un acide nucléique. Les quantités respectives de chaque composant peuvent être ajustées aisément par l'homme du métier en fonction du composé transfectant utilisé, de l'acide nucléique, et des applications recherchées (notamment du type de cellules à transfecter).

On entend au sens de l'invention par "acide nucléique" aussi bien un acide désoxyribonucléique qu'un acide ribonucléique. Il peut s'agir de séquences naturelles ou artificielles, et notamment d'ADN génomique (ADNg), d'ADN complémentaire (ADNc), d'ARN messager (ARNm), d'ARN de transfert (ARNt), d'ARN ribosomique (ARNr), de séquences hybrides telles que les chiméroplastes ADN/ARN, ou de séquences synthétiques ou semi-synthétiques, d'oligonucléotides modifiés ou non. Ces acides nucléiques peuvent être d'origine humaine, animale, végétale, bactérienne, virale, etc... Ils peuvent être obtenus par toute technique connue de l'homme du métier, et notamment par criblage de banques, par synthèse chimique, ou encore par des méthodes mixtes incluant la modification chimique ou enzymatique de séquences obtenues par criblage de banques. Ils peuvent être modifiés chimiquement. En général, ils contiennent au moins 10, 20, 50 ou 100 nucléotides consécutifs, et de préférence au moins 200 nucléotides consécutifs. Encore plus préférentiellement, ils contiennent au moins 500 nucléotides consécutifs.

Concernant plus particulièrement les acides désoxyribonucléiques, ils peuvent être simple ou double brin de même que des oligonucléotides courts ou des séquences plus longues. En particulier, les acides nucléiques sont avantageusement constitués par des plasmides, des vecteurs, des épisomes, des cassettes d'expression, etc... Ces acides désoxyribonucléiques peuvent porter une origine de réplication procaryote ou eucaryote fonctionnelle ou non dans la cellule cible, un ou plusieurs gènes marqueurs, des séquences régulatrices de la transcription ou de la réplication, des gènes d'intérêt thérapeutique, des séquences antisens modifiées ou non, des régions de liaison à d'autres composants cellulaires, etc...

De préférence, l'acide nucléique comprend un ou plusieurs gènes d'intérêt thérapeutique sous contrôle de séquences de régulation, par exemple un ou plusieurs promoteurs et un terminateur transcriptionnel actifs dans les cellules cibles.

Au sens de l'invention, on entend par gène d'intérêt thérapeutique notamment tout gène codant pour un produit protéique ayant un effet thérapeutique. Le produit protéique ainsi codé peut être notamment une protéine ou un peptide. Ce produit protéique peut être exogène homologue ou endogène vis-à-vis de la cellule cible, c'est-à-dire un produit qui est normalement exprimé dans la cellule cible lorsque celle-ci ne présente aucune pathologie. Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène d'intérêt thérapeutique peut aussi coder pour un mutant d'une protéine cellulaire, ayant une stabilité accrue, une activité modifiée, etc... Le produit protéique peut également être hétérologue vis-à-vis de la cellule cible. Dans ce cas, une protéine exprimée peut par exemple compléter ou apporter une activité déficiente dans la cellule, lui permettant de lutter contre une pathologie, ou stimuler une réponse immunitaire.

Parmi les produits thérapeutiques au sens de la présente invention, on peut citer plus particulièrement les enzymes, les dérivés sanguins, les hormones, les lymphokines et cytokines et leurs inhibiteurs ou leurs antagonistes (interleukines, interférons, TNF, les antagonistes de l'interleukine 1, les récepteurs solubles de l'interleukine 1 et TNFα, etc... : FR 92/03120), les facteurs de croissance, les neurotransmetteurs ou leurs précurseurs ou enzymes de synthèse, les facteurs trophiques (BDNF, CNTF, NGF, IGF, GMF, aFGF, bFGF, NT3, NT5, HARP/pléiotrophine, etc...) les apolipoprotéines (ApoAI, ApoAIV, ApoE, etc... : FR 93/05125), la dystrophine ou une minidystrophine (FR 91/11947), la protéine CFTR associée à la mucoviscidose, les gènes suppresseurs de tumeurs (p53, Rb, RaplA, DCC, k-rev, etc... : FR 93/04745), les gènes codant pour des facteurs impliqués dans la coagulation (Facteurs VII, VIII, IX), les gènes intervenant dans la réparation de l'ADN, les gènes suicides (thymidine kinase, cytosine déaminase), les gènes de l'hémoglobine ou d'autres transporteurs protéiques, les enzymes du métabolisme, catabolisme etc...

L'acide nucléique d'intérêt thérapeutique peut également être un gène ou une séquence antisens ou un ADN codant pour un ARN à fonction de ribozyme, dont l'expression dans la cellule cible permet de contrôler l'expression de gènes ou la transcription d'ARNm cellulaires. De telles séquences peuvent, par exemple, être transcrites dans la cellule cible en ARN complémentaires d'ARNm cellulaires et bloquer ainsi leur traduction en protéine, selon la technique décrite dans le brevet EP 140 308. Les gènes thérapeutiques comprennent également les séquences codant pour des ribozymes, qui sont capables de détruire sélectivement des ARN cibles (EP 321 201).

Comme indiqué plus haut, l'acide nucléique peut également comporter un ou plusieurs gènes codant pour un peptide antigénique, capable de générer chez l'homme ou l'animal une réponse immunitaire. Dans ce mode particulier de mise en oeuvre, l'invention permet la réalisation soit de vaccins soit de traitements immunothérapeutiques appliqués à l'homme ou à l'animal, notamment pour traiter ou prévenir des infections, par exemples virales ou bactériennes, ou des états cancéreux. Il peut s'agir notamment de peptides antigéniques spécifiques du virus d'Epstein Barr, du virus HIV, du virus de l'hépatite B (EP 185 573), du virus de la pseudo-rage, du "syncitia forming virus", d'autres virus ou encore de peptides antigéniques spécifiques de tumeurs (EP 259 212).

Préférentiellement, l'acide nucléique comprend également des séquences permettant l'expression du gène d'intérêt thérapeutique et/ou du gène codant pour le peptide antigénique dans la cellule ou l'organe désiré. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus. A cet égard, on peut citer par exemple les promoteurs des gènes E1A, MLP, CMV, RSV, etc... En outre, ces séquences d'expression peuvent être modifiées par addition de séquences d'activation, de régulation, etc... Il peut aussi s'agir de promoteur, inductible ou répressible.

Par ailleurs, l'acide nucléique peut également comporter, en particulier en amont du gène d'intérêt thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle. L'acide nucléique peut également comporter une séquence signal dirigeant le produit thérapeutique synthétisé vers un compartiment particulier de la cellule.

Les compositions selon l'invention peuvent en outre comporter un ou plusieurs adjuvants capables de s'associer aux complexes composé transfectant/acide nucléique et d'en améliorer le pouvoir transfectant. Dans un autre mode de mise en oeuvre, la présente invention concerne donc des compositions comprenant un acide nucléique, un composé transfectant tel que défini ci-avant et au moins un adjuvant capable de s'associer aux complexes composé transfectant/acide nucléique et d'en améliorer le pouvoir transfectant. Ces adjuvants qui permettent d'augmenter le pouvoir transfectant des composé selon la présente invention sont avantageusement choisis parmi les lipides (par exemple les lipides neutres, en particulier les phospholipides), les peptides (par exemple des dérivés d'histone, de nucléoline ou de protamine tels que ceux décrits dans WO 96/25508), les protéines (par exemple les protéines de type HMG telles que celles décrites dans WO 97/12051) et/ou les polymères (par exemple des polymères qui permettent de rendre les formulations composé transfectant/acide nucléique « furtives » tel que le polyéthylène glycol (PEG) introduit seul dans la formulation ou sous forme liée à un lipide afin de stabiliser colloïdalement les formulations composé transfectant/acide nucléique, par exemple le PEG-cholestérol). Dans cette optique, les compositions de l'invention peuvent comprendre à titre d'adjuvant, un ou plusieurs lipides neutres, qui possèdent notamment la propriété de former des agrégats lipidiques. Le terme « agrégat lipidique » est un terme générique qui inclus les liposomes de tous types (à la fois unilamellaires et multilamellaires) ainsi que les micelles ou encore des agrégats plus amorphes.

Plus préférentiellement, les lipides neutres utilisés dans le cadre de la présente invention sont des lipides à deux chaînes grasses. De manière particulièrement avantageuse, on utilise des lipides naturels ou synthétiques, zwitterioniques ou dépourvus de charge ionique dans les conditions physiologiques. Il peuvent être choisis plus particulièrement parmi la dioléoylphosphatidyléthanolamine (DOPE), l'oléoylpalmitoylphosphatidyléthanolamine (POPE), les distéaroyl, -palmitoyl, -mirystoylphosphatidyléthanolamines ainsi que leurs dérivé N-méthylés 1 à 3 fois, les phosphatidylglycérols, les diacylglycérols, les glycosyldiacylglycérols, les cérébrosides (tels que notamment les galactocérébrosides), les sphingolipides (tels que notamment les sphingomyélines) ou encore les asialogangliosides (tels que notamment les asialoGM1 et GM2). Selon une alternative particulièrement préférée, les adjuvants lipidiques utilisés dans le cadre de la présente invention sont choisis parmi DOPE, DOPC, le cholestérol, ou encore les dérivés lipidiques d'agents de surface non-ioniques tels que le PEG-cholestérol.

Ces différents lipides peuvent être obtenus soit par synthèse, soit par extraction à partir d'organes (par exemple le cerveau) ou d'oeufs, par des techniques classiques bien connues de l'homme du métier. En particulier, l'extraction des lipides naturels peut être réalisée au moyen de solvants organiques (voir également Lehninger, Biochemistry).

Préférentiellement, les compositions de l'invention comprennent de 0,01 à 20 équivalents d'adjuvant pour un équivalent d'acide nucléique en mol/mol et, plus préférentiellement, de 0,5 à 5 équivalents molaires.

Selon une autre alternative, les adjuvants cités ci-avant permettant d'améliorer le pouvoir transfectant des compositions selon la présente invention, notamment les peptides, les protéines ou certains polymères tels que le polyéthylène glycol, peuvent être conjugués aux composés transfectants selon l'invention, et non pas simplement mélangés. Notamment, le PEG peut être lié covalemment aux dérivés lipidiques d'aminoglycoside selon la présente invention soit au niveau d'une amine restante de l'aminoglycoside, soit au niveau des radicauxs hydroxyles de l'aminoglycoside.

Selon un mode de réalisation particulièrement avantageux, les compositions selon la présente invention comprennent en outre un élément de ciblage permettant d'orienter le transfert de l'acide nucléique. Cet élément de ciblage peut être un élément de ciblage extracellulaire permettant d'orienter le transfert de l'acide nucléique vers certains, types cellulaires ou certains tissus souhaités (cellules tumorales, cellules hépatiques, cellules hématopoïétiques...). Il peut également s'agir d'un élément de ciblage intracellulaire permettant d'orienter le transfert de l'acide nucléique vers certains compartiments cellulaires privilégiés (mitochondries, noyau etc...). L'élément de ciblage peut être mélangé aux composés transfectants selon l'invention et aux acides nucléiques, et dans ce cas l'élément de ciblage est de préférence lié covalemment à une chaîne aliphatique grasse (au moins 10 atomes de carbone) ou à un polyéthylène glycol. Selon une autre alternative, l'élément de ciblage est lié covalemment au composé transfectant selon l'invention, sur l'une des fonctions chimiques qui composent l'espaceur Y. Enfin, l'élément de ciblage peut également être lié à l'acide nucléique comme cela a été précisé précédemment.

Parmi les éléments de ciblage utilisables dans le cadre de l'invention, on peut citer les sucres, les peptides, les protéines, les oligonucléotides, les lipides, les neuromédiateurs, les hormones, les vitamines ou leurs dérivés. Préférentiellement, il s'agit de sucres, de peptides, de vitamines ou de protéines tels que par exemple des anticorps ou des fragments d'anticorps, des ligands de récepteurs cellulaires ou des fragments de ceux-ci, des récepteurs ou encore des fragments de récepteurs. Par exemple, il peut s'agir de ligands de récepteurs de facteurs de croissance, de récepteurs de cytokines, de récepteurs de type lectines cellulaires, de récepteurs au folate, ou de ligands à séquence RGD avec une affinité pour les récepteurs de protéines d'adhésion comme les intégrines. On peut également citer les récepteurs de la transferrine, des HDL et des LDL, ou le transporteur du folate. L'élément de ciblage peut également être un sucre permettant de cibler des lectines tels que les récepteurs aux asialoglycoprotéines ou aux syalydés tel que le Sialyl Lewis X, ou encore un fragment Fab d'anticorps, ou un anticorps simple chaîne (ScFv).

L'invention a également pour objet l'utilisation des composés transfectants selon la présente invention pour le transfert d'acides nucléiques dans les cellules *in vitro* ou *ex vivo*. Plus précisément, la présente invention a pour objet l'utilisation des composés transfectants tels que définis dans le cadre de l'invention pour la préparation d'un médicament destiné à traiter les maladies, en particulier les maladies qui résultent d'une déficience en un produit protéique ou nucléique. Le polynucléotide contenu dans ledit médicament code ledit produit protéique ou nucléique, ou constitue ledit produit nucléique, apte à corriger lesdites maladies *in vivo* ou *ex vivo.*

Pour des utilisations *in vivo*, par exemple en thérapie ou pour l'étude de la régulation de gènes ou la création de modèles animaux de pathologies, les compositions selon l'invention peuvent être formulées en vue d'administrations par voie topique, cutanée, orale, rectale, vaginale, parentérale, intranasale, intraveineuse, intramusculaire, sous-cutanée, intraoculaire, transdermique, intratrachéale, intrapéritonéale, etc... De préférence, les compositions de l'invention contiennent un véhicule pharmaceutiquement acceptable pour une formulation injectable, notamment pour une injection directe au niveau de l'organe désiré, ou pour une administration par voie topique (sur peau et/ou muqueuse). Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Les doses d'acides nucléiques utilisées pour l'injection ainsi que le nombre d'administrations peuvent être adaptées en fonction de différents paramètres, et notamment en fonction du mode d'administration utilisé, de la pathologie concernée, du gène à exprimer, ou encore de la durée du traitement recherchée. En ce qui concerne plus particulièrement le mode d'administration, il peut s'agir soit d'une injection directe dans les tissus, par exemple au niveau des tumeurs, soit d'une injection dans les voies circulatoires, soit encore d'un traitement de cellules en culture suivi de leur réimplantation *in vivo,* par injection ou greffe. Les tissus concernés dans le cadre de la présente invention sont par exemple les muscles, la peau, le cerveau, les poumons, la trachée, le foie, la rate, la moelle osseuse, le thymus, le coeur, la lymphe, le sang, les os, les cartilages, le pancréas, les reins, la vessie, l'estomac, les intestins, les testicules, les ovaires, le rectum, le système nerveux, les yeux, les glandes, les tissus conjonctifs, etc...

Un autre objet de la présente invention concerne une méthode in vitro ou ex vivo de transfert d'acides nucléiques dans les cellules comprenant les étapes suivantes :
(1) la mise en contact de l'acide nucléique avec un composé transfectant selon la présente invention, pour former un complexe, et
(2) la mise en contact des cellules avec le complexe formé en (1).

L'invention concerne en outre les composés de formule (I) pour utilisation dans une méthode de traitement du corps humain ou animal, ledit traitement comprenant les étapes suivantes :
(1) la mise en contact de l'acide nucléique avec un composé transfectant selon la présente invention, pour former un complexe, et
(2) la mise en contact des cellules du corps humain ou animal avec le complexe formé en (1).

La mise en contact des cellules avec le complexe peut être réalisée par incubation des cellules avec ledit complexe (pour des utilisations *in vitro* ou *ex vivo),* ou par injection du complexe dans un organisme (pour des utilisations *in vivo*)*.* D'une manière générale, la quantité d'acide nucléique destinée à être administrée dépend de très nombreux facteurs tels que par exemple la maladie à traiter ou à prévenir, la nature même de l'acide nucléique, la force du promoteur, l'activité biologique du produit exprimé par l'acide nucléique, de la condition physique de l'individu ou de l'animal (poids, âge ...), du mode d'administration et du type de formulation. En général, l'incubation est réalisée de préférence en présence de par exemple de 0,01 à 1000 µg d'acide nucléique pour 10⁶ cellules. Pour une administration *in vivo*, des doses d'acide nucléique allant de 0,01 à 50 mg peuvent par exemple être utilisées. L'administration peut être effectuée en dose unique ou répétée par intervalle.

Dans le cas où les compositions de l'invention contiennent en outre un ou plusieurs adjuvants tels que définis précédemment, le ou les adjuvants sont préalablement mélangés au composé transfectant selon l'invention et/ou à l'acide nucléique. Alternativement, le ou les adjuvants peuvent être administrés préalablement à 'administration des complexes nucléolipidiques.

Selon une autre alternative avantageuse, les tissus peuvent être soumis à un traitement chimique ou bien physique destiné à améliorer la transfection. Dans le cas du traitement physique, celui-ci peut utiliser des impulsions électriques comme dans le cas de l'électrotransfert, ou bien des forces mécaniques comme dans le cas de la sodoporation.

La présente invention fournit ains des composés pour utilisation dans une méthode particulièrement avantageuse pour le transfert d'acides *nucléiques in vivo,* notamment pour le traitement de maladies ledit traitement prenant l'administration *in vivo* ou *ex vivo* d'un acide nucléique codant pour une protéine ou pouvant être transcrit en un acide nucléique apte à corriger ladite maladie, ledit acide nucléique étant associé à un composé transfectant selon l'invention dans les conditions définies ci-avant.

Les composés transfectants de l'invention sont particulièrement utiles pour le transfert d'acides nucléiques dans des cellules primaires ou dans des lignées établies. Il peut s'agir de cellules fibroblastiques, musculaires, nerveuses (neurones, astrocytes, cellules gliales), hépatiques, hématopoïétiques (lymphocytes, CD34, dendritiques, etc...), épithéliales etc..., sous forme différenciée ou pluripotente (précurseurs).

Un autre objet de la présente invention concerne également les kits de transfection qui comprennent un ou plusieurs composés transfectants selon l'invention et leurs mélanges. De tels kits peuvent se présenter sous forme d'un emballage compartimenté de façon à recevoir différents récipients tels que par exemple des ampoules ou des tubes. Chacun de ces récipients comprend les différents éléments nécessaires pour effectuer la transfection, individuellement ou mélangés : par exemple un ou des composé(s) transfectant(s) selon l'invention, un ou des acide(s) nucléique(s), un ou des adjuvant(s), des cellules, etc...

### FIGURES

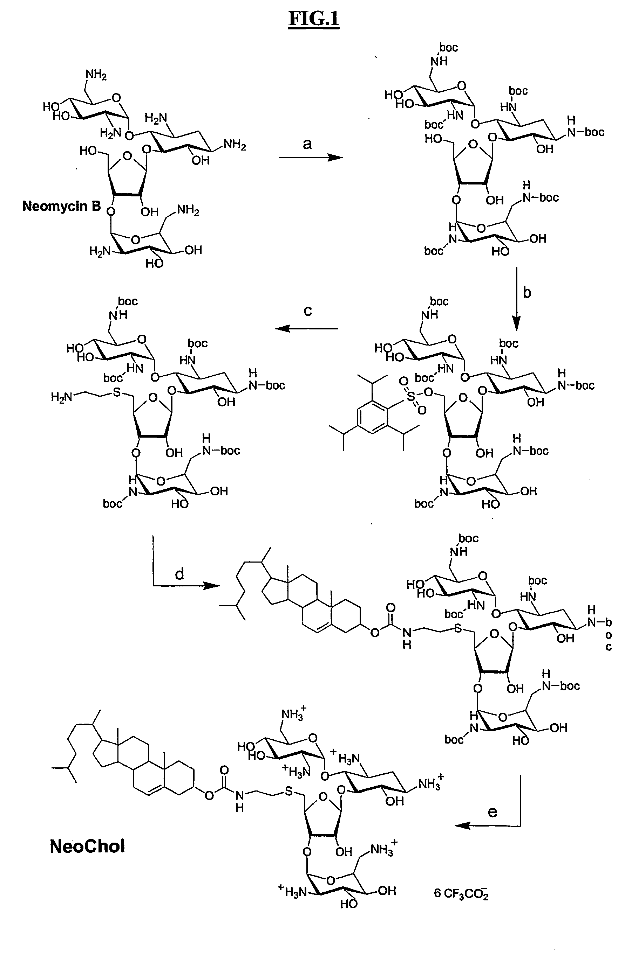

**Figure 1****:** Schéma réactionnel de la synthèse du trifluoroacétate de (5"-aminoethylsulfanyl) neomycin carbamoyl cholestérol (cf. exemple 3). a)Boc₂O Et₃N, DMF/H₂O, 60°C , 88%. b) Chlorure de 2,4,6-Triisopropylbenzenesulfonyle, Pyridine, 50%. c) HS-CH₂-CH₂-NH₂, NaOEt/EtOH, 46%. d) Chloroformiate de cholestéryle, Et₃N,THF/DMF, 52%. e) Acide trifluoroacétique, T<0°C, 40mn, 98%.

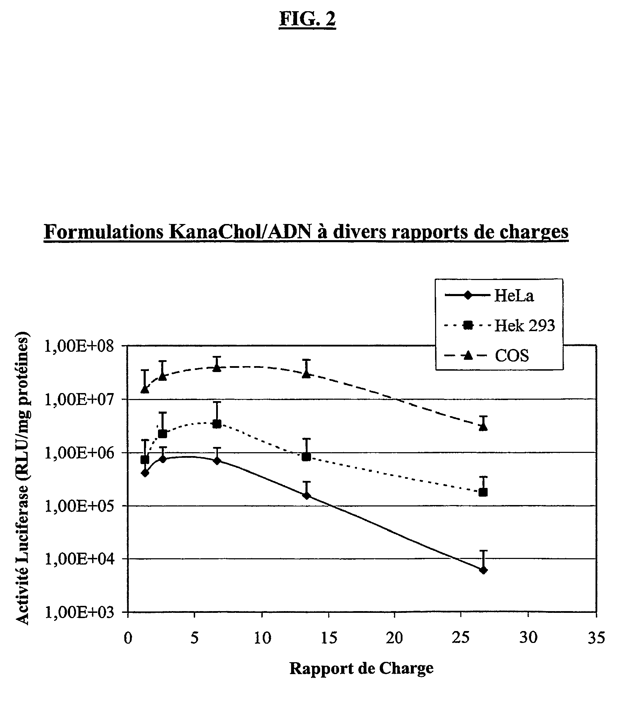

**Figure 2** **:** courbes dose-réponse de l'activité de transfection *in vitro* du composé transfectant « KanaChol ». L'expression du gène reporter est indiqué en fonction du rapport de charge des lipoplexes formé avec l'ADN plasmidique. Les cellules HeLa, HEK293 et COS ont été transfectées conformément au protocole décrit dans l'exemple 4 en utilisant des lipoplexes préparés en mélangeant 5 µg d'ADN plasmidique exprimant la luciférase avec la quantité requise de lipide. Les données sont exprimées en unité de lumière relative (« RLU ») par mg de protéines cellulaires.

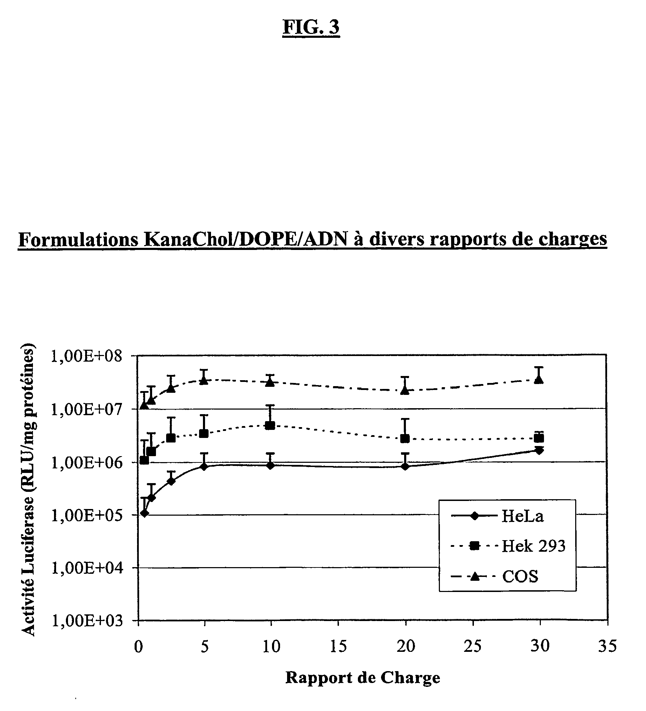

**Figure 3** **:** courbes dose-réponse de l'activité de transfection *in vitro* des liposomes KanaChol/DOPE (rapport molaire 1:1). L'expression du gène reporter est indiqué en fonction du rapport de charge des lipoplexes formé avec l'ADN plasmidique. Les cellules HeLa, HEK293 et COS ont été transfectées conformément au protocole décrit dans l'exemple 5 en utilisant des lipoplexes préparés en mélangeant 5 µg d'ADN plasmidique exprimant la luciférase avec la quantité requise de lipide pour former des lipoplexes caractérisés par le rapport de charge indiqué. Les données sont exprimées en unité de lumière relative (« RLU ») par mg de protéines cellulaires.

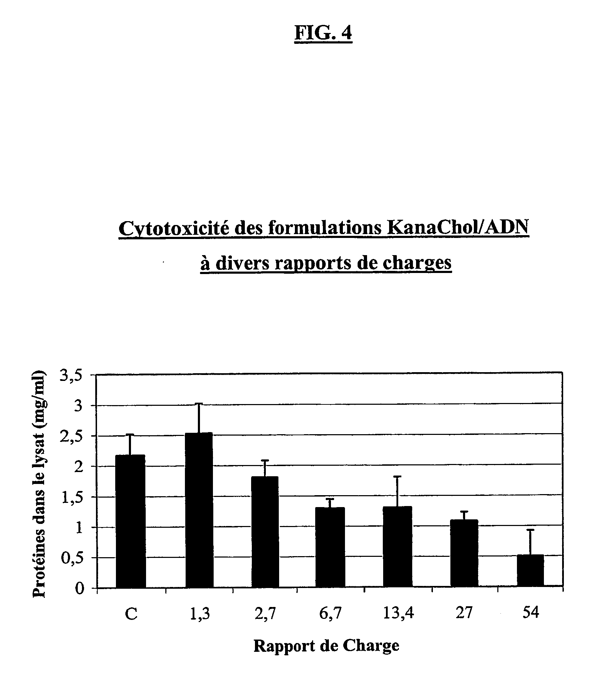

**Figure 4** **:** cytotoxicité du composé transfectant KanaChol. Des cellules HeLa ont été transfectées conformément au protocole décrit dans l'exemple 4 en utilisant des lipoplexes préparés en mélangeant 5 µg d'ADN plasmidique exprimant la luciférase avec la quantité requise de lipide pour former des lipoplexes caractérisés par le rapport de charge indiqué. A 48 heures post-transfection, les cellules ont été récupérées et la toxicité a été quantifiée en utilisant la quantité totale de protéines cellulaires dans le lysat cellulaire comme index du nombre de cellules, la mort cellulaire conduisant à une diminution des protéines extractibles. Le total des protéines cellulaires dans le lysat cellulaire est indiqué en fonction du rapport de charge des lipoplexes. Les données sont exprimées en concentration en protéines cellulaires extractibles dans le lysat cellulaire (à volume fixé).

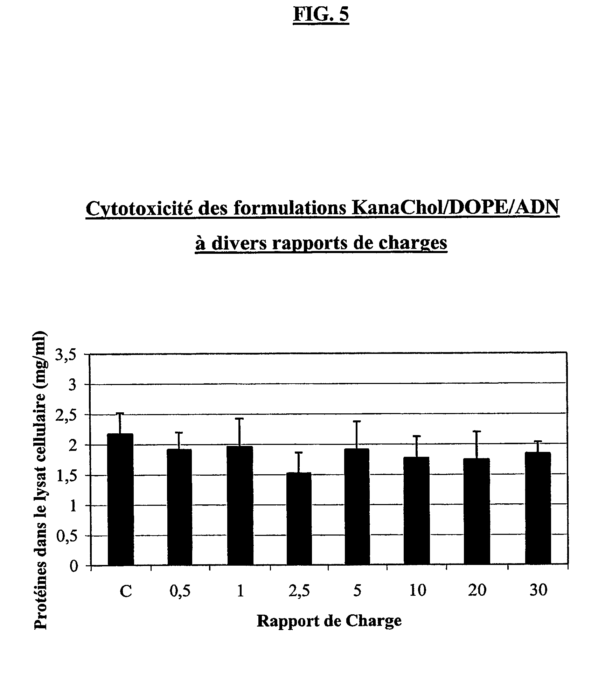

**Figure 5****:** cytotoxicité des liposomes KanaChol/DOPE (rapport molaire 1:1). Des cellules HeLa ont été transfectées conformément au protocole décrit dans l'exemple 5 en utilisant des lipoplexes préparés en mélangeant 5 µg d'ADN plasmidique exprimant la luciférase avec la quantité requise de lipide pour former des lipoplexes caractérisés par le rapport de charge indiqué. A 48 heures post-transfection, les cellules ont été récupérées et la toxicité a été quantifiée en utilisant la quantité totale de protéines cellulaires dans le lysat cellulaire comme index du nombre de cellules, la mort cellulaire conduisant à une diminution des protéines extractibles. Le total des protéines cellulaires dans le lysat cellulaire est indiqué en fonction du rapport de charge des lipoplexes. Les données sont exprimées en concentration en protéines cellulaires extractibles dans le lysat cellulaire (à volume fixé).

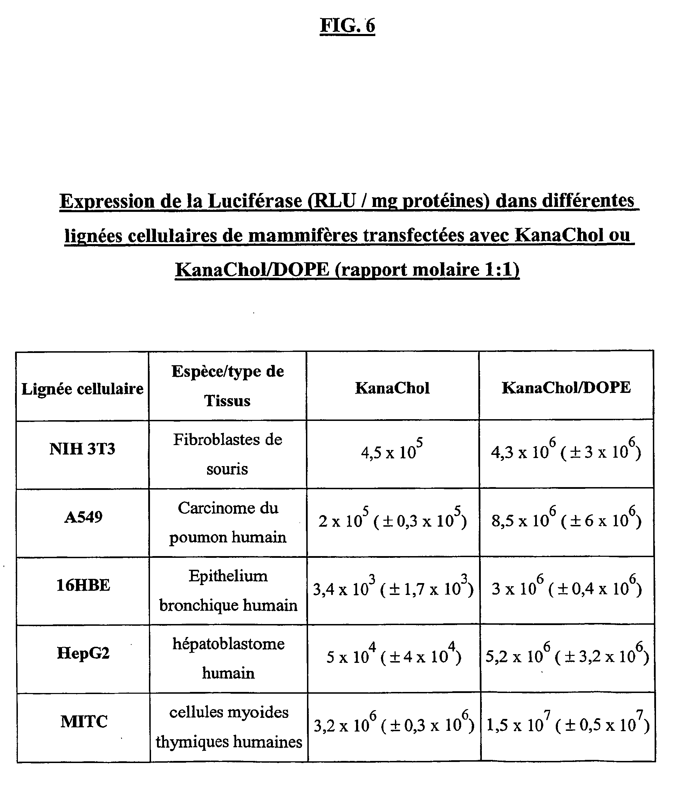

**Figure 6** **:** Expression de la luciférase dans différentes lignées cellulaires transfectées soit avec le composé transfectant KanaChol, soit avec les liposomes KanaChol/DOPE (1:1). Les différentes lignées cellulaires ont été transfectées conformément au protocole décrit dans l'exemple 6 en utilisant soit des lipoplexes KanaChol/ADN soit des lipoplexes KanaChol/DOPE/ADN (avec un rapport de charge positif 3-5) préparés en mélangeant 5 µg d'ADN plasmidique exprimant la luciférase et la quantité requise de lipide pour obtenir un rapport de charge positif 3-5. Les données sont exprimées en unité de lumière relative (« RLU ») par mg de protéines cellulaires.

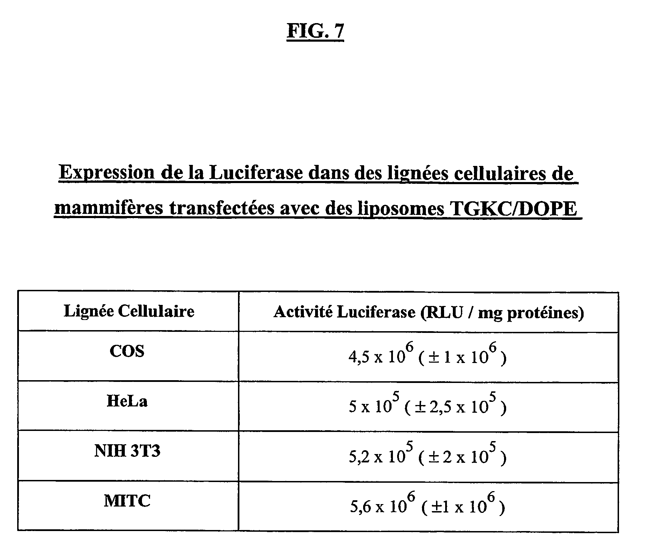

**Figure 7** (objet non revendiqué): Expression de la luciférase dans différentes lignées cellulaires transfectées avec les liposomes TGKC/DOPE (rapport molaire 1:1). Les différentes lignées cellulaires ont été transfectées conformément au protocole décrit dans l'exemple 7 en utilisant des lipoplexes TGKC/DOPE/ADN (avec un rapport de charge positif 3-5) préparés en mélangeant 5 µg d'ADN plasmidique exprimant la luciférase et la quantité requise de lipide pour obtenir un rapport de charge positif 3-5. Les données sont exprimées en unité de lumière relative (« RLU ») par mg de protéines cellulaires.

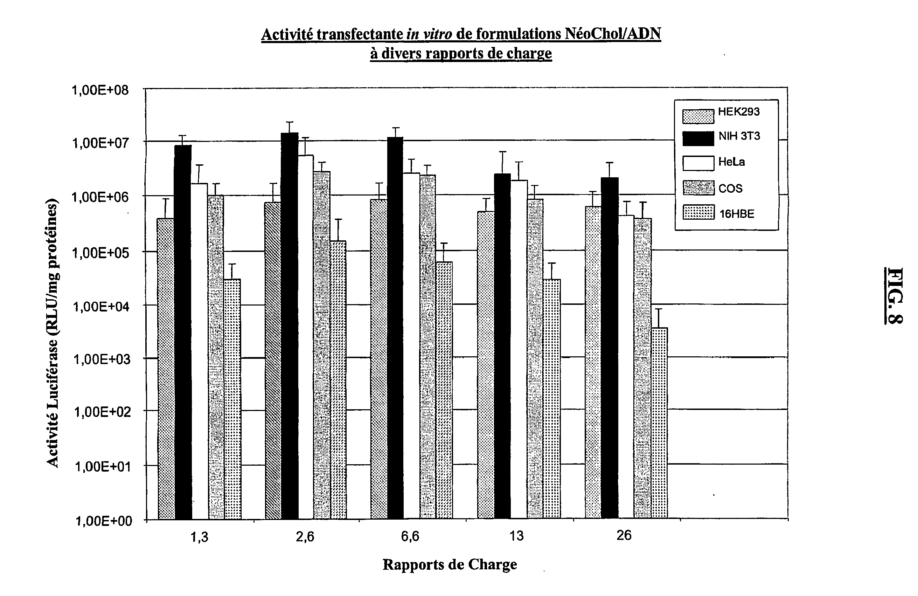

**Figure 8** **:** Expression de la luciférase dans différentes lignées cellulaires (HEK293, NIH 3T3, HeLa, COS, 16HBE) transfectées avec le composé transfectant NéoChol. L'expression du gène reporter est indiquée à différents rapports de charge des lipoplexes formé avec l'ADN plasmidique. Les différentes lignées cellulaires ont été transfectées conformément au protocole décrit dans l'exemple 8 en utilisant des lipoplexes NéoChol/ADN préparés en mélangeant 5 µg d'ADN plasmidique exprimant la luciférase. Les données sont exprimées en unité de lumière relative (« RLU ») par mg de protéines cellulaires.

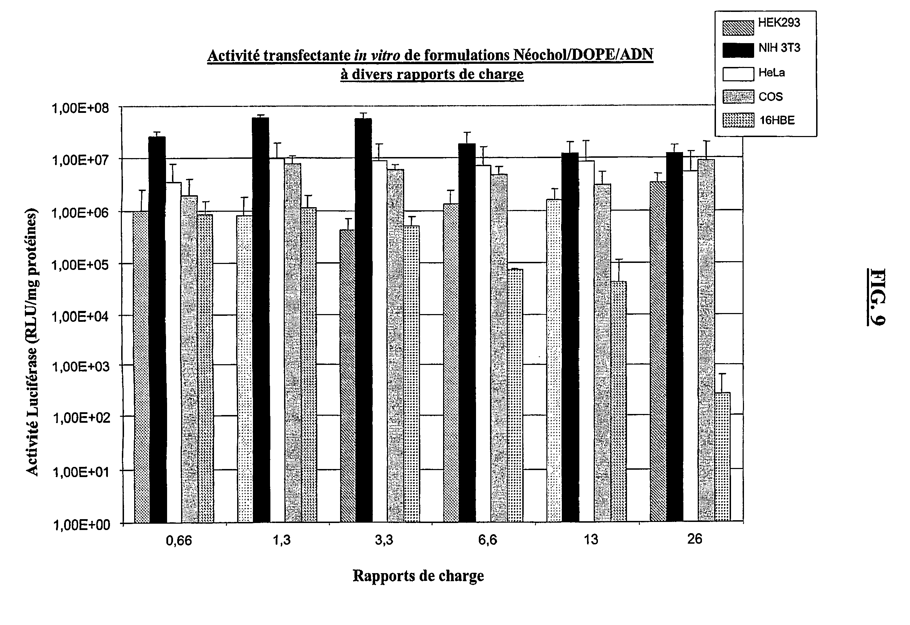

**Figure 9** : Expression de la luciférase dans différentes lignées cellulaires (HEK293, NIH 3T3, HeLa, COS, 16HBE) transfectées avec les liposomes NéoChol/DOPE (rapport molaire 1:1). L'expression du gène reporter est indiquée à différents rapports de charge des lipoplexes formé avec l'ADN plasmidique. Les différentes lignées cellulaires ont été transfectées conformément au protocole décrit dans l'exemple 8 en utilisant des lipoplexes NéoChol/DOPE/ADN préparés en mélangeant 5 µg d'ADN plasmidique exprimant la luciférase. Les données sont exprimées en unité de lumière relative (« RLU ») par mg de protéines cellulaires.

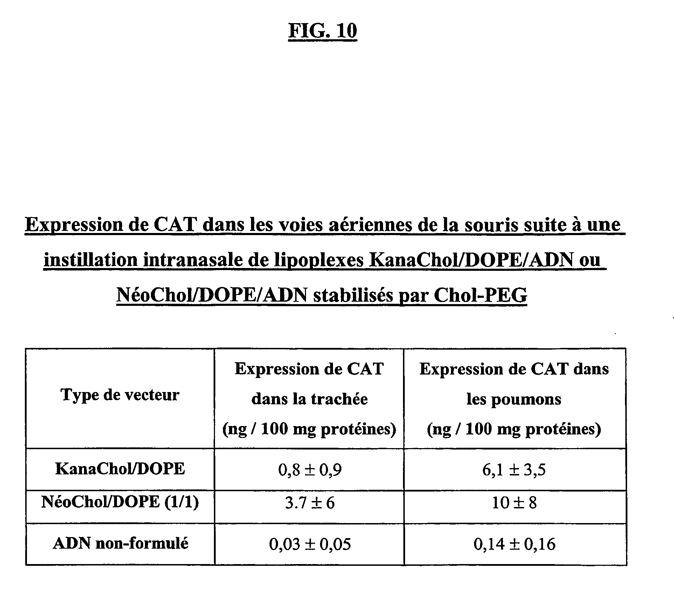

**Figure 10** **:** Expression du gène CAT dans les voies aériennes de la souris suite à l'instillation de lipoplexes KanaChol/DOPE/ADN stabilisés par du Chol-PEG (molécule hybride pour laquelle un polyéthylène glycol est lié covalemment au cholestérol) ou NéoChol/DOPE/ADN stabilisés par du Chol-PEG. Ces liposomes, caractérisés par un rapport de charge positif de 3,75 pour les lipoplexes KanaChol/DOPE/ADN stabilisé par du Chol-PEG ou 3,3 pour les lipoplexes NéoChol/DOPE/ADN stabilisé par du Chol-PEG et un rapport poids/poids en Cholestérol-PEG/ADN de 2, ont été utilisés pour délivrer une dose totale de 100 µg d'ADN plasmidique exprimant le gène CAT. Une instillation intranasale a été réalisée, puis la trachée et les poumons des souris traitées ont été prélevés afin de mesurer l'expression CAT selon la méthode décrite dans l'exemple 9. Les données sont exprimées en ng de protéine CAT /100 mg de protéines cellulaires totales.

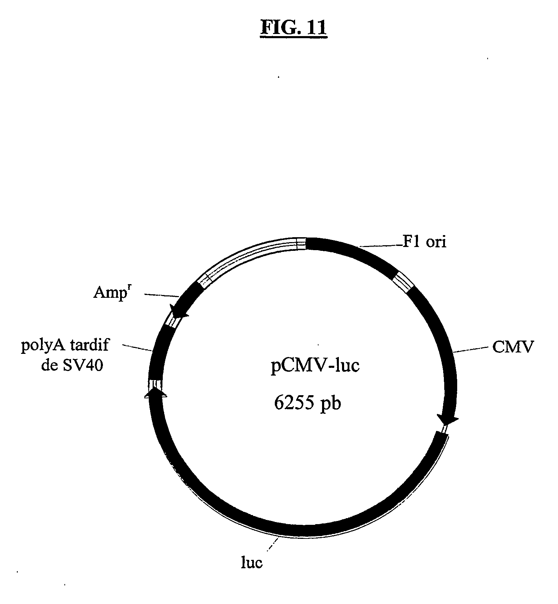

**Figure 11** **:** Représentation schématique du plasmide pCMV-Luc

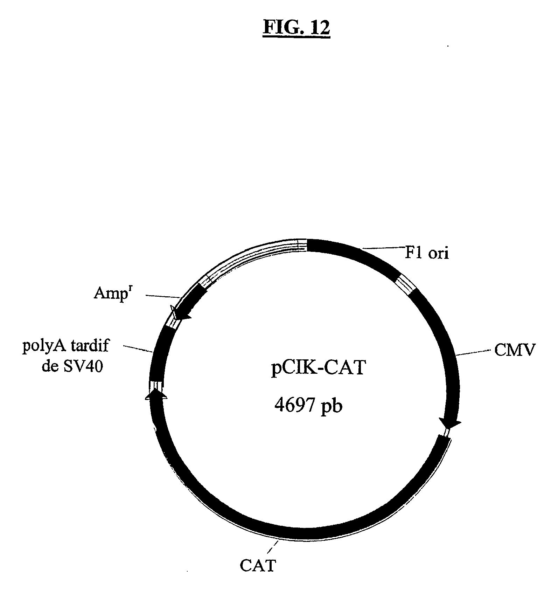

**Figure 12** **:** Représentation schématique du plasmide pCIK-CAT

### EXEMPLES

Les réactifs et catalyseurs tels que la triéthylamine, l'acide trifluoroacétique, l'acide p-toluène sulfonique, l'hexafluorophosphate de benzotriazol-1-yloxytripyrrolidinophosphonium (PyBOP), dicylclohexylcarbodiimide (DCC), le disulfure de carbone, le palladium sur charbon, la tétradécylamine, le di-*tert*-butyl dicarbonate, la 4-diméthylaminopyridine, la diisopropyléthylamine, le N-benzyloxycarbonyloxy-5-norbomene-2,3-dicarboximide, le carbonate de 2-(triméthylsilyl)éthyle p-nitrophényle, le cholestéryl chloroformiate sont disponibles commercialement, ainsi que la Kanamycin A. En outre, la N,N'-bis-tert-butoxycarbonylthiourée a été préparée conformément à la procédure publiée dans Synth. Commun. (1993), 23, pp. 1443-1445 et la néomycine amine a été préparée selon Tor et al. (J. Am. Chem. Soc. 2000, 122, 980-981).

Les spectres de Résonance Magnétique Nucléaire du Proton (RMN 1H) ont été enregistrés sur un spectromètres Bruker 200 MHz. Les déplacements chimiques sont exprimés en ppm (partie par million) et les multiplicités par les abréviations usuelles

Les plasmides utilisés sont :
- le pCMV-Luc décrit dans la publication de Patel et al. (BBRC, 2001, 6, pp. 536-543), qui contient le gène luc codant pour la luciférase sous contrôle du promoteur ultraprécoce CMV du cytomégalovirus. Ce plasmide est représenté à la figure 11. Il a été construit en sous-clonant le promoteur CMV (à partir du plasmide pcDNA3 de Invitrogen) en amont du gène luc dans le plasmide pGL2-Basic (Promega). Sa taille est de 6255 bp.
- le pCIK-CAT (pour les transfections *in vivo*) obtenu de D. Gill (Oxford, UK) et qui est décrit par Pitard et al. (J. Gene Med. 2001, 3, 478-487). Il a été construit en sous-clonant le gène CAT de E. Coli dans le plasmide pCI (Promega). Ce plasmide est représenté à la figure 12. Sa taille est de 4697 bp.

### Exemple 1: Synthèse du 3β[6'-kanamycin-carbamoyl]cholestérol (« KanaChol »)

### a) Synthèse de la [(1,3,3"-triamino)-6'-N-(benzyloxycarbonyl)lkanamycine A

A une solution de kanamycine A sous forme de base libre (8g ; 16,5 mmol) dans un mélange de diméthylsulfoxyde (800 ml) et d'eau (80 ml), on ajoute du N-benzyloxycarbonyloxy-5-norbomène-2,3-dicarboximide (5,18 g ; 16,5 mmol) et de la triéthylamine (1,67 g ; 16,5 mmol). Après une nuit sous agitation à température ambiante, un important précipité s'est formé et l'analyse du mélange réactionnel par CCM (chromatographie sur couche mince) révèle la présence de produits n'ayant pas réagi. On ajoute alors à nouveau de l'eau (640 ml) jusqu'à ce que le mélange devienne homogène, puis du N-benzyloxycarbonyloxy-5-norbornène-2,3-dicarboximide (2,6 g; 8,3 mmol). Après 4 jours d'agitation, les solvants sont évaporés sous vide et le résidu est chromatographié sur une colonne de silice avec un éluant variant d'un mélange dichlorométhane/méthanol/ammoniac (5/4/1) à un mélange méthanol/ammoniac (9/1). En évaporant les solvants des fractions concernées, on récupère le composé attendu (7,15 g ; 70 %).

### b) Synthèse de la [(1,3,3"-triaza-(triméthylsilyl-éthoxycarbonyl)]-[6'-N-(benzyloxycarbonyl)]kanamycine A (« Kana-tri[Teoc]-mono[Cbz] »)

A une solution de Kana-mono[Cbz] (0,5 g; 0,81 mmol) dans un mélange eau (7 ml)/dioxane (16 ml), on ajoute de la triéthylamine (0,25 g ; 3 équivalents ; 2,42 mmol) et du carbonate mixte de 2-(triméthylsilyl)éthyle et de p-nitrophényle (0,92 g ; 4 équivalents ; 3,23 mmol). Le mélange est agité à 55°C pendant 48 heures et, après concentration, la suspension obtenue est versée dans du butanol présaturé d'eau (60 ml). Après plusieurs lavages par une solution de soude 1N (800 ml) et par une solution saturée de chlorure de sodium, la phase butanolique est évaporée à sec. En ajoutant au résidu un mélange de méthanol et d'acétone et en concentrant la solution ainsi obtenue, on obtient le produit désiré sous forme d'une poudre jaune (0,73 g; 86 %).

### c) Synthèse de la [(1,3,3"-triaza-(triméthylsilyl-éthoxycarbonyl)]kanamycine A (« Kana-tri[Teoc]-NH₂ »)

A une solution dégazée de Kana-tri[Teoc]-mono[Cbz] (0,3 g ; 0,29 mmol) dans du méthanol, (25 ml), on ajoute du palladium sur charbon (Pd/C à 10 % ; 0,3 g) et on fait barboter un courant d'hydrogène dans le mélange pendant 1 heure. Celui-ci est encore agité pendant une nuit sous atmosphère d'hydrogène, puis la suspension est filtrée sur de la célite et évaporée à sec. On obtient ainsi le produit désiré sous forme d'un solide blanc (0,23 g; 90 %).

### d) Synthèse de la [(1,3,3"-triaza-(triméthylsilyl-éthoxycarbonyl)]-[6'-N-(3-oxocarbonyl cholestéryl)] kanamycine A (« Kana-tri[Teoc]-Chol »)

A une solution de Kana-tri[Teoc]-NH₂ (0,1 g; 0,11 mmol) dans un mélange de tétrahydrofuranne (5 ml) et de diméthylformamide (2 ml), on ajoute de la triéthylamine (1,5 équivalents; 0,017 g; 0,16 mmol) et du chloroformiate de cholestéryle (1,5 équivalents; 0,074 g; 0,16 mmol). On agite à la température ambiante pendant 48 heures avant de concentrer sous pression réduite jusqu'à ce que seul le diméthylformamide reste. En ajoutant de l'eau à cette solution, il se forme un précipité blanc que l'on filtre (0,12 g ; 83 %).

### e) Synthèse du trifluoroacétate de 1,3,3"-triamino-6'-N-(3-oxocarbonyl cholestéryl) kanamycine A (« Kanachol »)

Le Kana-tri[Teoc]-Chol obtenu à l'étape précédente (0,070 g; 0,053 mmol) est suspendu pendant 45 minutes dans de l'acide trifluoroacétique maintenu à 0°C dans un bain de glace. Après retour à la température ambiante, le mélange est concentré à sec. Le résidu est repris dans du méthanol et à nouveau concentré à sec. En répétant cette opération à plusieurs reprises, on obtient le Kanachol sous forme d'un solide blanc (0,063 g ; 97 %).

**RMN ¹H (CD₃OD)** : δ (ppm) 5,32 (m, 1H, H anomérique) ; 5,09 (m, 1H, H du cholestérol) ; 4,97 (m, 1H, H anomérique) ; 4,30 (m, 1H, H du cholestérol) ; 3,85-2,98 (m, 16H, divers protons des cycles glycosidiques) ; 2,30-0,81 (m, 44H, divers protons des cycles glycosidiques et du noyau cholestérol) ; 0,63 (s, 3H, CH₃ du cholestérol).

### Exemple de référence 2 : Synthèse du 3β[6'-(1,3,311-triguanidino)kanamycin-carbamoyl]-cholestérol (« TGKC »)

### a) Synthèse de la [1,3,3"-triaza-(N,N'-bis-tert-butoxycarbonyldiaminométhylène)-6'-N-(3-oxocarbonyl cholestéryl) kanamycine A (« Kana-tri[GuanBoc]-Chol »)

A une solution de Kanachol obtenu selon le protocole de l'exemple précédent (0,15 g ; 0,12 mmol) dans le diméthylformamide (10 ml), on ajoute successivement de la triéthylamine (30 équivalents ; 0,36 g ; 3,63 mmol), de la N,N'-bis-tert-butoxycarbonylthiourée (3,5 équivalents; 0,117 g; 0,42 mmol) et du chlorure mercurique (3,5 équivalents; 0,115 g; 0,42 mmol). Le mélange est agité à température ambiante et sous atmosphère inerte pendant 48 heures. La solution est alors versée dans un mélange d'eau et d'acétate d'éthyle et la phase aqueuse est extraite 4 fois à l'acétate d'éthyle. Après lavage avec une solution saturée de chlorure de sodium et séchage sur sulfate de sodium, les fractions organiques réunies sont concentrées à sec. Le produit souhaité est obtenu sous forme d'une poudre blanche (0,1 g; 51%)*.*

### b) Synthèse du sel de triflate de [1,3,3"-triaza-(N,N'-bis-diaminométhylène)-6'-N-(3-oxocarbonyl cholestéryl) kanamycine A (« Kana-tri[Guan]-Chol» ou « KTGC »).

Le Kana-tri[GuanBoc]-Chol obtenu à l'étape précédente (0,060 g ; 0,037 mmol) est mis en suspension dans de l'acide trifluoroacétique maintenu dans un bain de glace. Après 2,5 heures à 0°C puis retour à température ambiante, le mélange est concentré à sec. Le résidu rouge-brun est repris dans du méthanol puis concentré à nouveau à sec. Cette opération est renouvelée 3 fois. On ajoute alors du pentane et le mélange est soumis à une sonification de façon à obtenir une fine suspension qui est alors centrifugée. On récupère ainsi 0,030 g de KTGC pur (rendement : 60%).

**RMN ¹H (D₂O)** : δ (ppm) 5,48 (m, 1H, H anomérique) ; 5,05 (m, 1H) ; 3,97 (m, 1H, H du cholestérol) ; 3,70-2,60 (m, divers protons des cycles glycosidiques) ; 2,18-0,80 (m, divers protons des cycles glycosidiques et du noyau cholestérol).

### Exemple 3: Synthèse du (5"-aminoethylsulfanyl) néomycine carbamoyl cholestérol (« NéoChol »)

Le greffage de l'aminoglycoside Néomycine sur l'ensemble Y-L, se fait en utilisant une fonction amine provenant d'une modification réalisée selon Tor et al.(.J. Am. Chem. Soc. 2000, 122, 980-981). Le schéma réactionnel de la synthèse du NéoChol est présenté en figure 1.

### a) Synthèse du N- hexa terbutyloxycarbonyl NeoChol

A une solution de néomycine amine (102mg, 0,08 mmol, 1 équivalent), préparée selon Tor et al. (J. Am. Chem. Soc. 2000, 122, 980-981), dans un mélange de tétrahydrofuranne (6ml) et de diméthylformamide (2ml) on ajoute de la triéthylamine (14 ml ; 0,10mmol ; 1,2 équivalent) et du chloroformiate de cholestéryle (43,1 mg ; 0,10 mmol ; 1,2 equivalent). Le mélange réactionnel se trouble peu à peu et est agité durant une nuit. Les solvants volatils sont alors évaporés sous vide et le résidu est chromatographié sur une colonne de silice avec un mélange dichlorométhane /méthanol/ acétate d'éthyle 90/5/5 comme éluant. En évaporant les solvants des fractions concernées, on récupère le produit attendu sous forme d'un solide blanc.

Rf = 0,45 (Dichlorométhane/ Méthanol 9/1 ; chromatographie en couche mince)

**RMN ¹H (200 MHz, CDCl₃)**: δ (ppm) 6,15 (s élargi) ; 5,90 (m, 1 H); 5,35 (m, 2H) ; 5,36 (m, 1H, Chol); 5,25-4,76 (m, 5H) ; 4,60-3,20 (m, 15H) ; 3,10 (m, 1H) ; 2,86 (m, 2H) ; 2,69 (m, 2H) ; 2,50-1,72 (m, 10H) ; 1,72-0,84 (m, 87H); 0,71 (s, 3H, CH₃ Chol).

**Masse** (MALDI-TOF) : [NNa⁺] 1708,98.

### b) Synthèse du trifluoroacétate de (5"-aminoethylsulfanyl) neomycin carbamoylcholestérol

Le N-hexa terbutyloxycarbonyl NéoChol obtenu à l'étape précédente (60mg ; 0,035 mmol ; 1 équivalent) est suspendu dans de l'acide trifluoroacétique (2ml) placé dans un bain de glace. Après 50 minutes à 0°C, le mélange est concentré à sec. Le résidu est repris dans du méthanol puis concentré à nouveau à sec. Cette opération est renouvelée trois fois puis le résidu solide est dissous dans de l'eau et lyophilysé ; on récupère ainsi NéoChol sous forme d'une poudre blanche cotonneuse (59.6mg, 95.2%).

**RMN ¹H (200 MHz, [D₄]methanol)** : δ 6,05 (s élargi,1H); 5,46-5,31 (m, 3H); 4,43-4,29 (m, 4H); 4,21-3,82 (m, 5H); 3,67-3,11 (m, 13H); 2,89-2,60 (m, 3H); 2,58-2,25 (m, 3H); 2,20-1,73 (m, 7H); 1,72-0,84 (m, 33H); 0,71 (s, 3H, CH₃ Chol).

Masse (MALDI-TOF):[MH⁺] 1086,83 ; [MNa⁺] 1108,80.

### Exemple 4: Courbes dose-réponse de la transfection in vitro du composé transfectant KanaChol en milieu aqueux

Le but de cet exemple est d'illustrer la capacité des composés transfectants selon l'invention à transfecter des cellules *in vitro.*

Cette étude a été menée pour des lipoplexes comprenant différentes quantités de KanaChol : 6,6 ou 13,2 ou 33 ou 66 ou 132 nmoles de KanaChol pour 5 µg d'ADN. Afin de déterminer le rapport de charge moyen théorique, il a été considéré que 1 µg d'ADN représente 3 nmol de charges négatives et que 3 groupes amino de la kanamycine sont protonés à pH neutre lors de la formation des lipoplexes et de la transfection. En fait, des études de retard électrophorétique sur gel de la kanamycine complexée avec des acides nucléiques suggèrent que la charge positive moyenne du KanaChol serait plutôt de 2-3.

Préparation des lipoplexes KanaChol/ADN : le KanaChol obtenu selon l'exemple 1 est dissout dans une solution tampon Hepes à 20 mM (pH 7,4) à une concentration de 20 mM en charges positives (en considérant que 3 fonctions amino sont protonées à pH neutre). Puis, les lipoplexes sont préparés conformément au protocole décrit par Vigneron et al. (PNAS, 93, pp. 9682-9686, Sept. 1996) ou par Patel et al. (Biochem. and Biophys. Res. Comm., 281, pp. 536-543, 2001).

Culture des cellules : des cellules HeLa (American type Culture Collection (ATCC) Rockville, MD, USA) dérivées d'un carcinome d'épithélium cervical humain, sont mises en culture en présence d'un milieu de type MEM (« minimum essential medium ») supplémenté avec 10% de sérum de veau foetal et des antibiotiques. Le milieu et les additifs proviennent de Gibco/BRL life Technologies (Gaithersburg, MD,USA). Les cellules sont cultivées en flacons à 37°C et à 5 % de dioxyde de carbone en incubateur. La même procédure est utilisée pour les cellules HEK293 et COS.

Transfection : un jour avant la transfection, les cellules HeLa sont transférées dans des plaques de 6 puits avec un nombre de cellules d'environ 250 000 par puits. Ces dilutions représentent approximativement 80% de confluence après 24 heures.
Pour la transfection, les cellules sont lavées deux fois et incubées à 37°C avec 500 µl de milieu sans sérum. 500 µl de complexes contenant 5 µg d'ADN plasmidique sont ajoutés dans chaque puit (les complexes sont préparés au moins 30 minutes avant l'ajout dans les puits).
Après six heures à 37°C les plaques sans sérum sont complémentés avec 10 % (v/v) de SVF (« Sérum de Veau Foetal »).
L'ensemble des plaques est placé 24 heures à 37°C et à 5 % de dioxyde de carbone.

Ce protocole est analogue à ceux décrits par Vigneron et al. (PNAS, 93, pp. 9682-9686, Sept. 1996) ou par Patel et al. (Biochem. and Biophys. Res. Comm., 281, pp. 536-543,2001).

Détermination de l'activité luciférase : Brièvement, les cellules transfectées sont lavées deux fois avec 500 µl de PBS (tampon phosphate) puis lysées avec 250 µl de réactif (triphosphate 25 mM, pH 7,8 / MgCl₂ 8 mM / dithiothreitol 1 mM / glycérol 15 % / triton X-100 1 %). Le lysat est alors clarifié par centrifugation (15 minutes à 15°C) dans une microcentrifugeuse.
Un aliquote de 20 µl de surnageant du lysat centrifugé est dilué dans 100 µl de tampon de lyse, auquel on ajoute 4 µl d'ATP 25 mM (Sigma) et 20 µl de luciférine 25 mM (Sigma) . Les échantillons sont alors placés dans un luminomètre Lumet LB9501 (Berthold, Nashua, NH) et la valeur d'intégration de l'émission de lumière est mesurée pendant 10 secondes.
L'activité luciférase est ainsi exprimée en Unité de Lumière Relative (« RLU » : «Relative light unit ») et normalisée avec la concentration de protéines de l'échantillon obtenue par l'utilisation d'un kit Biorad.

Ce protocole est analogue à celui décrit par Vigneron et al. (PNAS, 93, pp. 9682-9686, Sept. 1996) ou par Patel et al. (Biochem. and Biophys. Res. Comm., 281, pp. 536-543, 2001).

Les résultats pour les 3 lignées cellulaires résumés dans la Figure 2 montrent des taux d'expression élevés avec une efficacité de transfection optimale pour les lipoplexes ayant un rapport de charge moyen théorique d'environ 5. La transfection est la plus élevée dans la lignée cellulaire COS.

Un contrôle a également été réalisé avec des complexes formés entre la kanamycine (non-liée à un dérivé de cholestérol) et l'ADN : aucune transfection n'a été observée.

Ces données démontrent donc que le KanaChol permet d'effectuer une transfection efficace de l'ADN dans les cellules *in vitro.* Ces résultats sont également cohérents avec l'hypothèse que la transfection est liée à l'interaction électrostatique entre les lipoplexes positifs et la surface cellulaire globalement négative.

La figure 2 montre également que l'efficacité de transfection diminue pour des rapports de charge très élevés (supérieurs à environ 7). Cette diminution pourrait refléter un certain effet toxique des lipides cationiques en général, et donc du KanaChol en particulier, lorsqu'il sont utilisés en grande quantité.

Ainsi, la cytotoxicité du KanaChol a également été mesurée à différents rapports de charges avec l'ADN. La toxicité a été quantifiée en utilisant la quantité totale de protéines cellulaires dans le lysat cellulaire par puit comme index du nombre de cellules. Une diminution de la quantité totale de protéines cellulaires dans le lysat cellulaire par puit correspond à la diminution du nombre de cellules et témoigne par conséquent de l'existence d'une certaine toxicité des lipoplexes KanaChol/ADN vis-à-vis des cellules.

Détermination du niveau de protéine : la détermination du niveau de protéine a été effectuée en utilisant le kit Biorad selon un protocole identique à celui décrit par Oudrhriri et al. (PNAS, 94, pp. 1651-1656, 1997), et en se conformant aux instructions du fabricant.

Les résultats résumés dans la figure 4 montrent une diminution du nombre de protéines cellulaires extractibles total à rapport de charges élevé, c'est-à-dire une augmentation de la cytotoxicité parallèlement à l'augmentation des concentrations en KanaChol. Ainsi, la diminution de l'activité de transfection à rapports de charges élevés dans les 3 lignées cellulaires testées pourrait être liée à l'existence d'une certaine toxicité du KanaChol utilisé en très grandes quantités.

### Exemple 5: Courbes dose-réponse de la transfection in vitro des liposomes KanaChol/DOPE

Le but de cet exemple est d'illustrer la capacité des composés transfectants selon l'invention à transfecter des cellules *in vitro* lorsqu'ils sont formulés sous forme de liposomes avec des lipides neutres.

Fréquemment, les lipides cationiques sont formulés sous forme de liposomes avec du lipide neutre DOPE. On pense en effet que l'utilisation de la DOPE, du fait de ses propriétés fusogéniques, aiderait la transfection en facilitant la sortie des lipoplexes des vésicules endocytiques dans lesquelles ils sont internalisés. Il a ainsi été montré par Felgner et al. (J. Biol. Chem., 1994, 269, pp. 2550-2561) pour une série de lipides cationiques que leur formulation avec 50% de DOPE permettait d'augmenter l'efficacité de transfection de 2 à 5 fois par rapport à des formulations sans lipide zwitterionique.

C'est pourquoi des liposomes KanaChol/DOPE (1 1 molaire) ont été préparés de la façon suivante : un mélange de KanaChol et de DOPE (rapport molaire 1 :1) dans du chloroforme est évaporé sous vide et resuspendu dans une solution tampon Hepes à 20 mM (pH 7,4). La concentration finale en lipides est de 5 mg/ml. Le mélange est ensuite soniqué pendant 10 minutes en utilisant un appareil à ultrasons Branson Sonifier 450 équipé d'un terminal Sonifier Cell Disruptor B-30, pour obtenir des liposomes. La solution résultante est refroidie à température ambiante avant filtration à travers un filtre de 0,22 µm (Millex GS, Millipore).

De la même façon qu'à l'exemple précédent, cette étude a été menée pour des liposomes comprenant différentes quantités de KanaChol de façon à mesurer l'efficacité de transfection à rapports de charges moyens théoriques variables, dans les 3 lignées cellulaires HeLa, HEK293 et COS.

Les résultats résumés dans la figure 3 montrent des niveaux d'expression élevés de la luciférase pour les 3 lignées cellulaires. Ces niveaux de transfection sont tout à fait comparables à ceux obtenus avec les formulations de KanaChol seul (sans DOPE), ce qui pourrait s'expliquer par le fait que les 3 lignées cellulaires utilisées sont très facilement transfectables. En revanche, on observe qu'il n'y a pas de diminution claire de l'activité de transfection à rapports de charges élevés lorsqu'on utilise des liposomes KanaChol/DOPE/ADN.

Ainsi, la cytotoxicité des liposomes KanaChol/DOPE a également été mesurée à différents rapports de charges avec l'ADN, selon un protocole analogue à celui décrit à l'exemple précédent. Les données résumées à la figure 5 indiquent que ces formulations liposomales ne présentent pas de toxicité apparente à rapports de charges élevés. Il apparaît donc que le KanaChol, utilisé en formulation liposomale, est un vecteur de transfection à la fois efficace et non toxique.

### Exemple 6: Spectres de l'activité de transfection in vitro du composé transfectant KanaChol et des liposomes KanaChol/DOPE

Le but de cet exemple est d'illustrer la capacité des composés transfectants selon l'invention à transfecter d'autres types cellulaires *in vitro,* qu'ils soient utilisés seuls ou sous forme de liposome avec de la DOPE.

Les expériences de transfection *in vitro* menées dans les exemples 4 et 5 ont à nouveau été effectuées de manière similaire, mais avec d'autres lignées cellulaires moins usuelles (l'espèce et la spécificité de chaque lignée cellulaire sont indiquées à la Figure 6). Les formulations KanaChol/ADN et KanaChol/DOPE/ADN sont utilisées à des rapports de charges compris entre 3 et 5 (valeurs optimales telles que définies aux exemples 4 et 5). Les résultats obtenus sont résumés dans le tableau de la figure 6.

Il apparaît que la formulation KanaChol/DOPE (rapport molaire 1 :1) induit une efficacité de transfection élevée pour toutes les lignées cellulaires, le KanaChol utilisé seul (sans DOPE) étant globalement moins efficace, en particulier dans les lignées cellulaires 16HBE et HepG2. Il semble donc que l'utilisation de la DOPE aurait un effet bénéfique sur l'efficacité de transfection.

### Exemple de référence 7 : Activité de transfection in vitro des liposomes TGKC/DOPE

Le but de cet exemple est d'illustrer la capacité des composés transfectants polyguanidylés selon l'invention (formulés sous forme de liposomes) à transfecter des cellules *in vitro.*

Des formulations TGKC/DOPE (rapport molaire 1 :1) obtenues de façon analogue au protocole décrit dans l'exemple 5 sont ainsi utilisées pour transfecter *in vitro* différentes lignées cellulaires (COS, HeLa, NIH3T3 et MITC), selon des méthodes de transfection similaires à celles décrites dans les exemples précédents. Le rapport de charge des lipoplexes TGKC/DOPE/ADN a été fixé entre 3 et 5, c'est-à-dire à des valeurs qui s'étaient révélées optimales précédemment avec le KanaChol. Naturellement, afin de déterminer le rapport de charges moyen théorique, il a été considéré que les 3 groupes guanidiniums du TGKC sont chargés positivement à pH neutre (du fait du pKa élevé des fonctions guanidiniums).

Les résultats résumés à la figure 7 montrent clairement que les liposomes TGKC/DOPE permettent de transfecter efficacement les différentes lignées cellulaires testées.

### Exemple 8 : Spectres de l'activité de transfection in vitro du composé transfectant NéoChol et des liposomes NéoChol/DOPE

Le but de cet exemple est d'illustrer la capacité des composés transfectants selon l'invention à transfecter différents types cellulaires *in vitro,* qu'ils soient utilisés seuls ou sous forme de liposome avec de la DOPE.

Les expériences de transfection *in vitro* menées dans les exemples 4, 5, 6 et 7 ont été effectuées de manière similaire en utilisant les formulations NéoChol/ADN et NéoChol/DOPE/ADN à différents rapports de charges (compris entre 1,3 et 26 pour NéoChol/ADN et entre 0,66 et 26 pour NéoChol/DOPE/ADN) et dans une série de 5 lignées cellulaires (HeLa, NIH 3T3, HEK293, COS et 16HBE). Les lipoplexes et les liposomes ont été préparés de manière analogue au protocole décrit dans les exemples 4 et 5 (en estimant que 5 fonctions amino sont protonées pour NéoChol à pH neutre). Les résultats obtenus sont résumés en figure 8 et 9.

Les résultats résumés à la figure 8 montrent clairement que les formulations NéoChol/ADN permettent de transfecter efficacement les différentes lignées cellulaires testées.

Il apparaît que la formulation NéoChol/DOPE (rapport molaire 1 :1) induit une efficacité de transfection élevée pour toutes les lignées cellulaires, le NéoChol utilisé seul (sans DOPE) semblant globalement un peu moins efficace, en particulier dans la lignée cellulaire 16HBE (sauf pour les rapports de charges élevés). Il apparait donc que l'utilisation de la DOPE aurait un effet bénéfique sur l'efficacité de transfection.

### Exemple 9 : Transfert de mènes in vivo dans les voies aériennes de la souris par les liposomes KanaChol/DOPE/Chol-PEG et NéoChol/DOPE/Chol-PEG

Cet exemple illustre la capacité des composés transfectants selon l'invention à transfecter l'ADN dans des cellules *in vivo.*

Le transfert de gène *in vivo* a été effectué avec 50 µl (par dose) de milieu transfectant contenant les lipoplexes KanaChol/DOPE/ADN ou NéoChol/DOPE/ADN stabilisés par du Chol-PEG dans les voies aériennes de souris femelles BALB/c ayant un poids de 30 g (administration intranasale). Chaque animal a reçu 3 doses à des intervalles d'environ 4 heures, soit une quantité totale de 100 µg d'ADN plasmidique pCIK-CAT.

Les formulations utilisées ont été préparées de la façon suivante : des liposomes KanaChol/DOPE sont préparés selon le protocole décrit dans l'exemple 6. Puis on ajoute une solution de stabilisant stérique Chol-PEG (un cholestérol lié à une chaîne de polyéthylène glycol contenant environ 100 unités d'oxyéthylène) immédiatement avant le mélange avec l'ADN plasmidique pCIK-CAT en solution aqueuse. La concentration en ADN est ainsi d'approximativement 0,6 mg/ml et le rapport de charges positif est de 3,75. Le rapport final Chol-PEG/ADN est alors de 2 (poids/poids). L'utilisation de Chol-PEG permet l'obtention de solutions stables colloïdalement, ce qui permet d'avoir une efficacité de transfection très élevée dans les voies aériennes de la souris *in vivo* par instillation intranasale des formulations liposomales. Les formulations de NéoChol/DOPE/Chol-PEG sont préparés de la même manière, mais le rapport de charge positif utilisé est de 3,3 pour ces formulations.

Le gène reporter utilisé dans le cas présent est le gène CAT (« *Escherichia Coli* chloramphénicol acetyltransferase »). L'administration dans les voies aériennes est effectuée par instillation intranasale. 48 heures post-transfection, les animaux sont tués et les poumons et la trachée sont prélevés séparément pour analyse. Les morceaux de tissus sont placés dans un tampon TEN (40 mM de Tris-HCI, 1 mM d'EDTA et 150 mM de chlorure de sodium, pH de 7,8) et agités sur de la glace pendant environ 30 secondes en utilisant un agitateur Ultrax-Turrax T25 (Fisher Bioblock Scientific, Strasbourg, France). Les cellules sont ensuite lysées par 3 cycles gel-dégel et un supemageant clair est obtenu par centrifugation. La concentration en CAT est déterminée en utilisant un essai CAT ELISA effectué conformément aux instruction du fabricant (Boehringer Mannheim). Les niveaux de CAT sont exprimés en ng de protéine CAT pour 100 mg de protéines totales, la concentration en protéines étant déterminée en utilisant un essai Bio-Rad.

Le tableau de la figure 10 présente les niveaux d'expression du gène CAT obtenus dans les homogénats de trachée et de poumon. L'expression du gène CAT est la plus élevée dans le poumon. La figure 10 indique également que des niveaux très bas d'expression sont obtenus après administration de doses identiques d'ADN « nu » (c'est-à-dire non-formulé). Ces résultats suggèrent que dans les conditions de l'expérience, les liposomes KanaChol/DOPE et NéoChol/DOPE permettent une efficacité de transfection *in vivo,* dans les voies respiratoires de la souris, supérieure à celle obtenue avec de l'ADN nu. Les niveaux d'expression obtenus grâce au liposomes NéoChol/DOPE sont du même ordre de grandeur que ceux obtenus grâce aux liposomes KanaChol/DOPE. Ces résultats sont également tout à fait analogues avec ceux qui avaient été obtenus avec des formulations BGTC/DOPE (décrites dans la demande WO 97/31935), formulations qui s'étaient révélées tout particulièrement efficaces et intéressantes pour la transfection des voies respiratoires (Densmore et al., J. of Gene Med., 1999, Vol 1 (4)).

## Revendications

1. Composé transfectant constitué d'un aminoglycoside relié à un lipide par l'intermédiaire d'un espaceur, de formule générale (I) :
A-Y-L (I)
dans laquelle :
- A représente un aminoglycoside choisi parmi l'amikacine, l'arbékacine, la dibékacine, la gentamicine, l'isépamycine, la kanamycine, la mieronomycine, la paromomycine, la néomycine, ou la tobramycine,
- Y représente un espaceur, et
- L représente
• soit un radical -(R1)R2 avec R1 et R2 qui représentent, indépendamment l'un de l'autre, un atome d'hydrogène ou une chaîne • aliphatique grasse ou R1 ou R2 est absent, étant entendu que l'un au moins de R1 et de R2 représente une chaîne aliphatique grasse choisie parmi les radicaux alkyle contenant 14, 16, 18, 20 ou 22 atomes de carbone et une, deux ou trois insaturations,
• soit un radical -N-R3 ou -O-R3, avec R3 qui représente un composé polycyclique de type cholestane,
le cas échéant sous forme d'isomère, de mélange ou de sel.

2. Composé transfectant selon la revendication 1 **caractérisé en ce que** ledit aminoglycoside est choisi parmi la kanamycine, la paromomycine, la néomycine, ou la tobramycine.

3. Composé transfectant selon les revendications 1 ou 2 **caractérisé en ce que** ledit espaceur comprend une ou plusieurs fonctions chimiques choisies parmi les alkyles ayant 1 à 6 atomes de carbone, les fonctions cétone, ester, éther, amino, amide, amidine, carbamate, thiocarbamate, le glycérol, l'urée, la thiourée, ou les cycles aromatiques.

4. Composé transfectant selon la revendication 3 **caractérisé en ce que** ledit espaceur est choisi parmi les radicaux de formule :
-C(O)-
-NH-C(O)-CH₂-CH₂-
-W-(CH₂-)ₖ-W'-
ou encore :
-(CH₂-)ᵢ-W-(CH₂)ⱼ
dans laquelle i, j et k sont des entiers choisis entre 1 et 6 inclus et W et W' sont des groupes identiques ou différents choisis parmi les fonctions cétone, ester, éther, amino, amide, amidine, carbamate, thiocarbamate, le glycérol, l'urée, la thiourée, ou encore les cycles aromatiques,

5. Composés transfectant selon l'une des revendications précédentes, **caractérisé en ce que** ledit composé polycyclique de type cholestane est choisi parmi le cholestérol, le cholestanol, le 3-α-5-cyclo-5- α-cholestan-6-β-ol, l'acide cholique, le cholestérylformiate, le chotestanylformiate, le 3α,5-cyclo-5α -cholestan-6β-yl formiate, la cholestérylamine, la 6-(1,5-diméthylhexyl)-3a,5a-diméthylhexadécahydrocyclopenta[a]cyclopropa[2,3]-cyclo-penta[1,2-f]naphtalèn-10-ylamine, ou la cholestanylamine.

6. Composé transfectant selon l'une des revendications précédentes **caractérisé en ce qu'**il s'agit du 3 β [6'-kanamycin-carbamoyl]cholestérol.

7. Composition **caractérisée en ce qu'**elle comprend un composé transfectant selon l'une des revendications 1 à 6, et un acide nucléique.

8. Composition selon la revendication 7 **caractérisée en ce que** l'acide nucléique est un acide désoxyribonucléique ou bien un acide ribonucléique.

9. Composition selon la revendication 7 ou 8 **caractérisée en ce que** ledit acide nucléique comprend un ou plusieurs gènes d'intérêt thérapeutique sous contrôle de séquences de régulation.

10. Composition selon les revendications 7 à 9 **caractérisée en ce que** ledit acide nucléique est un gène ou une séquence antisens.

11. Composition selon l'une des revendications 7 à 10 **caractérisée en ce qu'**elle comprend en outre un ou plusieurs adjuvants.

12. Composition selon la revendication 11 **caractérisée en ce que** ledit ou lesdit adjuvant(s) sont choisis parmi les lipides, les peptides, les protéines et/ou les polymères.

13. Composition selon la revendication 12 **caractérisée en ce que** ledit ou lesdits adjuvant(s) sont choisis parmi les lipides neutres.

14. Composition selon la revendication 13 **caractérisée en ce que** ledit lipide neutre est choisi parmi les lipides naturels ou synthétiques, zwitterioniques ou dépourvus de charge ionique dans les conditions physiologiques.

15. Composition selon la revendication 14 **caractérisée en ce que** ledit lipide neutre est choisi parmi la dioléoylphosphatidyléthanolamine (DOPE), l'oléoylpalmitoyl-phosphatidyléthanolamine (POPE), les di-stéaroyl, -palmitoyl, - mirystoyl phosphatidyléthanolamines ainsi que leurs dérivé N-méthylés 1 à 3 fois, les phosphatidylglycérols, les diacylglycérols, les glycosyldiacylglycérols, les cérébrosides (tels que notamment les galactocérébrosides), les sphingolipides (tels 10 que notamment les sphingomyélines), les asialogangliosides (tels que notamment les asialoGMI et GM2), la DOPC et le cholestérol.

16. Composition selon la revendication 12 **caractérisée en ce que** ledit adjuvant est le polyéthylène glycol (PEG) ou le PEG-Cholestérol.

17. Composition selon l'une des revendications 7 à 16 **caractérisée en ce qu'**elle comprend en outre un élément de ciblage extracellulaire ou intracellulaire.

18. Composition selon la revendication 17 **caractérisée en ce que** ledit élément de ciblage est choisi parmi les sucres, les peptides, les protéines, les oligonucléotides, les lipides, les neuromédiateurs, les hormones, les vitamines ou leurs dérivés.

19. Composition selon les revendications 17 ou 18 **caractérisée en ce que** ledit élément de ciblage est lié covalemment à une chaîne alkyle grasse contenant au moins 10 atomes de carbone ou à un polyéthylène glycol (PEG).

20. Composition selon la revendication 17 **caractérisée en ce que** ledit élément de ciblage est lié covalemment soit au composé transfectant selon l'une des revendications 1 à 6, sur l'une des fonctions chimiques qui composent l'espaceur Y soit à l'acide nucléique.

21. Composition selon l'une des revendications 7 à 20 **caractérisée en ce qu'**elle comprend en outre un véhicule pharmaceutiquement acceptable pour une formulation injectable.

22. Composition selon l'une des revendications 7 à 20 aractérisée en ce qu'elle comprend en outre un véhicule pharmaceutiquement acceptable pour une administration sur la peau et/ou les muqueuses.

23. Utilisation d'un composé transfectant selon l'une des revendications 1 à 6 pour le transfert d'acides nucléiques in vitro.

24. Utilisation d'un composé transfectant selon l'une des revendications 1 à 6 pour fabriquer un médicament destiné à soigner les maladies.

25. Méthode de transfert in vitro ou ex vivo d'acides nucléiques dans les cellules **caractérisée en ce qu'**elle comprend les étapes suivantes :
(1) la mise en contact de l'acide nucléique avec un composé transfectant tel que défini dans les revendications 1 à 6 pour former un complexe, et
(2) la mise en contact des cellules avec le complexe formé en (1).

26. Méthode de transfert d'acides nucléiques dans les cellules selon la revendication 25 **caractérisée en ce que** ledit agent de transfert et/ou ledit acide nucléique sont préalablement mélangés à un ou plusieurs adjuvant(s) et/ou à un élément de ciblage tels que définis dans les revendications 12 à 19.

27. Méthode de transfert d'acides nucléiques dans les cellules selon la revendication 25 **caractérisée en ce que** un ou plusieurs adjuvants tels que définis aux revendications 12 à 16 sont préalablement administrés aux cellules.

28. Méthode de transfert d'acides nucléiques dans les cellules selon l'une des revendications 25 à 27 **caractérisée en ce que** les cellules sont en outre soumises à un traitement chimique ou physique.

29. Kit de transfection **caractérisé en ce qu'**il comprend un ou plusieurs composés transfectants selon l'une des revendications 1 à 6 et leurs mélanges.

## Claims

1. Transfection compound made up of an aminoglycoside bound to a lipid by means of a spacer, of general formula (I):
A-Y-L (I)
wherein:
- A represents an aminoglycoside chosen from the group comprising amikacin, arbekacin, dibekacin, gentamicin, isepamicin, kanamycin, micronomicin, paromomycin, neomycin or tobramycin,
- Y represents a spacer, and
- L represents:
• either a radical -(R1)R2, R1 and R2 representing, independently from one another, a hydrogen atom or a fatty aliphatic chain, or R1 or R2 is absent, it being understood that at least one of R1 and R2 represent a fatty aliphatic chain chosen in the group consisting of alkyl radicals containing 14, 16, 18, 20 or 22 carbon atoms and one, two, or three unsaturations.
• or a radical -N-R3 or -O-R3, R3 representing a polycyclic compound of the cholestane type,
in the form of an isomer, mixture or salt, depending on the case.

2. Transfection compound according to claim 1, **characterized in that** said aminoglycoside is chosen from the group consisting of kanamycin, paromomycin, neomycin, or tobramycin.

3. Transfection compound according to claims 1 or 2, **characterized in that** said spacer comprises one or more chemical functions chosen from an alkyl group having 1 to 6 carbon atoms, ketone-, ester-, ether-, amino-, amide-, amidine-, carbamate-, thiocarbamate-function compounds glycerol, urea, thiourea, or aromatic rings.

4. Transfection compound according to claim 3, **characterized in that** said spacer is chosen from the group consisting of radicals from the formula:
-C(O)-
-NH-C(O)-CH₂-CH₂-
-W- (CH₂)ₖ-W'-
or:
- (CH₂)ᵢ-W-(CH₂)ⱼ
wherein i, j and k are integer numbers between 1 and 6 inclusively, and W and W' are identical or different groups chosen from a group comprising ketone-, ester, ether, amino, amide, amidine, carbamate, thiocarbamate, function compounds, glycerol, urea, thiourea, or even aromatic rings.

5. Transfection compounds according to any one of the preceding claims, **characterized in that** said polycyclic compound of the cholestane type is chosen from the group comprising cholesterol, cholestanol, 3-α-5-cyclo-5-α-cholestan-6-β-ol, cholic acid, cholesteryl formiate, cholestanyl formiate, 3-α-5-cyclo-5-α-cholestan-6-β-yl formiate, cholesteryl amine, 6-(1,5-dimethylhexyl)-3a,5a-dimethylhexadecahydrocyclopenta [a]cyclopropa[2,3]-cyclo-penta[1,2-f]naphtalen-10-ylamine, or cholestanyl amine.

6. Transfection compound according to any one of the preceding claims, **characterized in that** it is a 3β[6'-kanamycin-carbamoyl]cholesterol.

7. Composition **characterized in that** it comprises a transfection compound according to any one of claims 1 to 6, and a nucleic acid.

8. Composition according to claim 7, **characterized in that** the nucleic acid is a deoxyribonucleic acid or a ribonucleic acid.

9. Composition according to claim 7 or 8, **characterized in that** said nucleic acid comprises one or more therapeutically interesting genes under the control of regulatory sequences.

10. Composition according to claims 7 to 9, **characterized in that** said nucleic acid is a gene or an antisense sequence.

11. Composition according to any one of claims 7 to 10, **characterized in that** it also comprises one or more additives.

12. Composition according to claim 11, **characterized in that** said additive or additives are chosen from the group consisting of lipids, peptides, proteins and/or polymers.

13. Composition according to claim 12, **characterized in that** said additive or additives are chosen from the group consisting of neutral lipids.

14. Composition according to claim 13, **characterized in that** said neutral lipid is chosen from the group consisting of natural or synthetic lipids, zwitterions or lipids without ionic charge under physiological conditions.

15. Compositions according to claim 14, **characterized in that** said neutral lipid is chosen from the group consisting of dioleoylphosphatidylethanolamine (DOPE), oleoylpalmitoyl-phosphatidylethanolamine (POPE), distearoyl, palmitoyl, myristoyl phosphatidylethanolamines as well as their derivatives N-methylated 1 to 3 times, phosphatidylglycerols, diacylglycerols, glycosyl diacylglycerols, cerebrosides (such as, notably, galactocerebrosides), sphingolipids, (such as, notably, sphingomyelins), asialogangliosides (such as, notably, asialo GM1 and GM2), DOPC and cholesterol

16. Composition according to claim 12, **characterized in that** said additive is polyethylene glycol (PEG) or PEG-cholesterol.

17. Composition according to any one of claims 7 to 16, **characterized in that** it further comprises an element for extracellular or intracellular targeting.

18. Composition according to claim 17, **characterized in that** said targeting element is chosen from the group consisting of sugars, peptides, proteins, oligonucleotides, lipids, neuromediators, hormones, vitamins or their derivatives.

19. Composition according to claims 17 or 18, **characterized in that** said targeting element is covalently bound to a fatty alkyl chain containing at least 10 carbon atoms or to a polyethylene glycol (PEG).

20. Compositions according to claim 17, **characterized in that** said targeting element is covalently bound either to a transfection compound according to one of claims 1 to 6, on one of the chemical functions that make up spacer Y, or to a nucleic acid.

21. Composition according to any one of claims 7 to 20, **characterized in that** it further comprises a pharmaceutically acceptable excipient for an injectable formulation.

22. Composition according to any one of claims 7 to 20, **characterized in that** it further comprises a pharmaceutically acceptable excipient for administration on the skin or mucosa.

23. Use of a transfection compound according to any one of claims 1 to 6 for transferring nucleic acids *in vitro.*

24. Use of a transfection compound according to any one of claims 1 to 6 to manufacture a drug intended for treating patients.

25. Method for *in vitro* or ex vivo nucleic acid transfer in cells, **characterized in that** it comprises the following steps:
(1) connecting the nucleic acid with a transfection compound, as defined in claims 1 to 6, to form a complex, and
(2) connecting the cells with the complex formed in step (1).

26. Nucleic acid transfer method in cells according to claim 25, **characterized in that** said transfer agent and/or said nucleic acid are mixed beforehand with one or more additive(s) and/or a targeting element as defined in claims 12 to 19.

27. Nucleic acid transfer method in cells according to clam 25, **characterized in that** one or more additives, as defined in claims 12 to 16, are administered to the cells beforehand.

28. Nucleic acid transfer method in cells according to any one of claims 25 to 27, **characterized in that** the cells are further subjected to a chemical or physical treatment.

29. Transfection kit **characterized in that** it comprises one or more transfection compounds according to claims 1 to 6 and their mixtures.

## Patentansprüche

1. Transfizierende Verbindung, die aus einem Aminoglycosid besteht, das über einen Abstandshalter mit einem Lipid verbunden ist, der allgemeinen Formel (I):
A-Y-L (I)
in der:
- A ein Aminoglycosid darstellt, das ausgewählt ist aus Amikacin, Arbekacin, Dibekacin, Gentamicin, Isepamycin, Kanamycin, Micronomycin, Paromomycin, Neomycin oder Tobramycin,
- Y einen Abstandshalter darstellt und
- L darstellt:
• entweder einen Rest -(R1)R2, wobei R1 und R2 unabhängig voneinander ein Wasserstoffatom oder eine aliphatische Fettkette darstellen oder wobei R1 oder R2 abwesend ist, vorausgesetzt, dass mindestens eines von R1 und von R2 eine aliphatische Fettkette darstellt, die aus Alkylresten ausgewählt ist, die 14, 16, 18, 20 oder 22 Kohlenstoffatome und 1, 2 oder 3 Unsättigungen enthalten,
• oder einen Rest -N-R3 oder -O-R3, wobei R3 eine polycyclische Verbindung vom Cholestan-Typ darstellt,
gegebenenfalls in Form eines Isomers, einer Mischung oder eines Salzes.

2. Transfizierende Verbindung nach Anspruch 1, **dadurch gekennzeichnet, dass** das Aminoglycosid aus Kanamycin, Paromomycin, Neomycin oder Tobramycin ausgewählt ist.

3. Transfizierende Verbindung nach Anspruch 1 oder 2, **dadurch gekennzeichnet, dass** der Abstandshalter eine oder mehrere chemische Funktionen umfasst, die ausgewählt sind aus Alkylen mit 1 bis 6 Kohlenstoffatomen, Keton-, Ester-, Ether-, Amino-, Amid-, Amidin-, Carbamat-, Thiocarbamat-Funktionen, Glycerol, Harnstoff, Thioharnstoff oder aromatischen Zyklen.

4. Transfizierende Verbindung nach Anspruch 3, **dadurch gekennzeichnet, dass** der Abstandshalter ausgewählt ist aus den Resten der Formel:
-C(O)-
-NH-C(O)-CH₂-CH₂-
-W-(CH₂-)ₖ-W'-
oder auch:
-(CH₂-)ᵢ-W-(CH₂)ⱼ
in der i, j und k ganze Zahlen sind, die zwischen 1 und 6 einschließlich ausgewählt sind, und W und W' identische oder verschiedene Gruppen sind, die aus Keton-, Ester-, Ether-, Amino-, Amid-, Amidin-, Carbamat-, Thiocarbamat-Funktionen, Glycerol, Harnstoff, Thioharnstoff oder auch aromatischen Zyklen ausgewählt sind.

5. Transfizierende Verbindungen nach einem der vorangehenden Ansprüche, **dadurch gekennzeichnet, dass** die polycyclische Verbindung vom Cholestan-Typ ausgewählt ist aus Cholesterol, Cholestanol, 3-α-5-Cyclo-5-α-cholestan-6-β-ol, Cholsäure, Cholesterylformiat, Cholestanylformiat, 3-α-5-Cyclo-5-α-cholestan-6-β-ylformiat, Cholesterylamin, 6-(1,5-Dimethylhexyl)-3a,5a-di-methylhexadecahydrocyclopenta[a]cyclopropa[2,3]cyclopenta[1,2,f]naphthalin-10-ylamin oder Cholestanylamin.

6. Transfizierende Verbindung nach einem der vorangehenden Ansprüche, **dadurch gekennzeichnet, dass** es sich um 3β-[6'-Kanamycincarbamoyl]-cholesterol handelt.

7. Zusammensetzung, **dadurch gekennzeichnet, dass** sie eine transfizierende Verbindung nach einem der Ansprüche 1 bis 6 und eine Nucleinsäure umfasst.

8. Zusammensetzung nach Anspruch 7, **dadurch gekennzeichnet, dass** die Nucleinsäure eine Desoxyribonucleinsäure oder auch eine Ribonucleinsäure ist.

9. Zusammensetzung nach Anspruch 7 oder 8, **dadurch gekennzeichnet, dass** die Nucleinsäure ein oder mehrere therapeutisch interessierende Gene unter der Kontrolle von Regulationssequenzen umfasst.

10. Zusammensetzung nach den Ansprüchen 7 bis 9, **dadurch gekennzeichnet, dass** die Nucleinsäure ein Gen oder eine Antisense-Sequenz ist.

11. Zusammensetzung nach einem der Ansprüche 7 bis 10, **dadurch gekennzeichnet, dass** sie ferner ein oder mehrere Adjuvantien umfasst.

12. Zusammensetzung nach Anspruch 11, **dadurch gekennzeichnet, dass** das Adjuvans oder die Adjuvantien ausgewählt sind aus Lipiden, Peptiden, Proteinen und/oder Polymeren.

13. Zusammensetzung nach Anspruch 12, **dadurch gekennzeichnet, dass** das Adjuvans oder die Adjuvantien aus neutralen Lipiden ausgewählt sind.

14. Zusammensetzung nach Anspruch 13, **dadurch gekennzeichnet, dass** das neutrale Lipid aus natürlichen oder synthetischen Lipiden ausgewählt ist, die unter physiologischen Bedingungen zwitterionisch oder frei von ionischer Ladung sind.

15. Zusammensetzung nach Anspruch 14, **dadurch gekennzeichnet, dass** das neutrale Lipid ausgewählt ist aus Dioleoylphosphatidylethanolamin (DOPE), Oleoylpalmitoylphosphatidylethanolamin (POPE), Distearoyl-, -palmitoyl-, -myristoylphosphatidylethanolaminen sowie ihren 1- bis 3-fach N-methylierten Derivaten, Phosphatidylglycerolen, Diacylglycerolen, Glycosyldiacylglycerolen, Cerebrosiden (wie insbesondere den Galactocerebrosiden), Sphingolipiden (wie insbesondere den Sphingomyelinen), Asialogangliosiden (wie insbesondere den AsialoGM1 und -GM2), DOPC und Cholesterol.

16. Zusammensetzung nach Anspruch 12, **dadurch gekennzeichnet, dass** das Adjuvans Polyethylenglycol (PEG) oder PEG-Cholesterol ist.

17. Zusammensetzung nach einem der Ansprüche 7 bis 16, **dadurch gekennzeichnet, dass** sie ferner ein extrazelluläres oder intrazelluläres Targeting-Element enthält.

18. Zusammensetzung nach Anspruch 17, **dadurch gekennzeichnet, dass** das Targeting-Element ausgewählt ist aus Zuckern, Peptiden, Proteinen, Oligonucleotiden, Lipiden, Neuromediatoren, Hormonen, Vitaminen oder deren Derivaten.

19. Zusammensetzung nach Anspruch 17 oder 18, **dadurch gekennzeichnet, dass** das Targeting-Element kovalent mit einer Fettalkylkette, die mindestens 10 Kohlenstoffatome enthält, oder mit einem Polyethylenglycol (PEG) verbunden ist.

20. Zusammensetzung nach Anspruch 17, **dadurch gekennzeichnet, dass** das Targeting-Element kovalent entweder über eine der chemischen Funktionen des Abstandshalters Y mit der transfizierenden Verbindung nach einem der Ansprüche 1 bis 6 oder mit der Nucleinsäure verbunden ist.

21. Zusammensetzung nach einem der Ansprüche 7 bis 20, **dadurch gekennzeichnet, dass** sie ferner ein pharmazeutisch annehmbares Vehikel für eine injizierbare Formulierung umfasst.

22. Zusammensetzung nach einem der Ansprüche 7 bis 20, **dadurch gekennzeichnet, dass** sie ferner ein pharmazeutisch annehmbares Vehikel für eine Verabreichung auf der Haut und/oder den Schleimhäuten umfasst.

23. Verwendung einer transfizierenden Verbindung nach einem der Ansprüche 1 bis 6 für den Transfer von Nucleinsäuren in vitro.

24. Verwendung einer transfizierenden Verbindung nach einem der Ansprüche 1 bis 6 zur Herstellung eines Medikaments, das dazu bestimmt ist, Krankheiten zu behandeln.

25. In-vitro- oder ex-vivo-Verfahren zum Transfer von Nucleinsäuren in Zellen, **dadurch gekennzeichnet, dass** es die folgenden Schritte umfasst :
(1) In-Kontakt-Bringen von Nucleinsäure mit einer transfizierenden Verbindung, wie in den Ansprüchen 1 bis 6 definiert, um einen Komplex zu bilden, und
(2) In-Kontakt-Bringen von Zellen mit dem Komplex, der in (1) gebildet wurde.

26. Verfahren zum Transfer von Nucleinsäuren in Zellen nach Anspruch 25, **dadurch gekennzeichnet, dass** das Transfermittel und/oder die Nucleinsäure zuvor mit einem oder mehreren Adjuvantien und/oder mit einem Targeting-Element, wie in den Ansprüchen 12 - 19 definiert, gemischt werden.

27. Verfahren zum Transfer von Nucleinsäuren in Zellen nach Anspruch 25, **dadurch gekennzeichnet, dass** zuvor ein oder mehrere Adjuvantien, wie in den Ansprüchen 12 - 16 definiert, den Zellen verabreicht werden.

28. Verfahren zum Transfer von Nucleinsäuren in Zellen nach einem der Ansprüche 25 - 27, **dadurch gekennzeichnet, dass** die Zellen ferner einer chemischen oder physikalischen Behandlung unterzogen werden.

29. Transfektions-Kit, **dadurch gekennzeichnet, dass** es eine oder mehrere transfizierende Verbindungen nach einem der Ansprüche 1 bis 6 und deren Mischungen umfasst.
